Europäisches Patentamt

European Patent Office

Office européen des brevets

(19)

(11) Veröffentlichungsnummer : **0 514 413 B1**

(12) **EUROPÄISCHE PATENTSCHRIFT**

(45) Veröffentlichungstag der Patentschrift :
04.05.94 Patentblatt 94/18

(21) Anmeldenummer : 91903270.6

(22) Anmeldetag : 08.02.91

(86) Internationale Anmeldenummer :
PCT/DE91/00106

(87) Internationale Veröffentlichungsnummer :
WO 91/12269 22.08.91 Gazette 91/19

(51) Int. Cl.⁵ : **C07K 7/00,** C07K 13/00,
G01N 33/569, G01N 33/68,
C07K 3/18, C07K 3/22,
C07K 3/20, C12N 15/10

(54) **IMMUNOLOGISCH AKTIVE PEPTIDE BZW. POLYPEPTIDE DES PARVOVIRUS B19.**

Die Akte enthält technische Angaben, die nach dem Eingang der Anmeldung eingereicht wurden und die nicht in dieser Patentschrift enthalten sind.

(30) Priorität : 08.02.90 DE 4003826

(43) Veröffentlichungstag der Anmeldung :
25.11.92 Patentblatt 92/48

(45) Bekanntmachung des Hinweises auf die Patenterteilung :
04.05.94 Patentblatt 94/18

(84) Benannte Vertragsstaaten :
AT BE CH DE DK ES FR GB IT LI LU NL SE

(56) Entgegenhaltungen :
WO-A-88/02026

(56) Entgegenhaltungen :
**Chemical Abstracts, vol. 114, No. 3, published 21 January 1991 (21.01.91), (Columbus, Ohio, USA), F.B. Rayment et al. "The production of human parvovirus capsid proteins in Escherichia coli and their potential as diagnostic antigens", see page 203, right-hand column, abstract No. 18 831d; J.Gen. Virol. 1990, 71(11), 265-72**
**Chemical Abstracts, vol. 109, No. 17, published 24 October 1988 (24.10.88) (Columbus, Ohio, USA), K. Ozawa et al. "Translational regulation of B19 parvovirus capsid protein production by multiple upstream AUG triplets", see page 177, left-hand column, abstract No. 143 557s; J. Biol. Chem. 1988, 263 (22), 10922-6**

(73) Patentinhaber : **MIKROGEN MOLEKULARBIOLOGISCHE ENTWICKLUNGS-GMBH**
**Westendstrasse 125/G**
**D-80339 München (DE)**

(72) Erfinder : **SOUTSCHEK, Erwin**
**Schäftlarnstrasse 126**
**D-8000 München 70 (DE)**
Erfinder : **MOTZ, Manfred**
**Schachnerstrasse 7**
**D-8000 München 70 (DE)**

(74) Vertreter : **Keller, Günter, Dr. et al**
**Lederer, Keller & Riederer Patentanwälte**
**Prinzregentenstrasse 16**
**D-80538 München (DE)**

EP 0 514 413 B1

## Beschreibung

Das humane Parvovirus B19 (im folgenden kurz: B19) wurde 1975 zufällig in Plasmaproben von Blutspendern (Cossart, Y.E., Field, A.M., Cant, B., Widdows, D.: Parvovirus-like particles in human sera. Lancet I (1975) 72-73) mittels Gegenstromelektrophorese entdeckt. In den letzten Jahren wurde gezeigt, daß B19 bei Patienten mit chronisch-hämolytischer Krise eine aplastische Krise verursachen kann und das ätiologische Agens des Erythema infectiosum (EI) ist.

B19 weist elektronenmikroskopisch eine Größe von ca. 20nm auf. Die Partikel haben eine ikosaedrische Symmetrie. Neben den Viruspartikeln zeigen sich auch "leere" Kapside, die keine DNA enthalten. Die Dichte in $CsCl_2$ beträgt 1,36 - 1,40g/ml. Das Virusgenom besteht aus einer einzelsträngigen DNA von 5,4kb. Die Nukleotidsequenz des Genoms eines Parvovirus B19 wurde von einem Klon abgeleitet, der beinahe das vollständige virale Genom enthielt (R.O. Shade et al. Journal of Virology (1986) S.921).In jedes Viruspartikel wird jeweils nur ein DNA-Strang von entweder Plus- oder Minusorientierung verpackt. B19 ist ein autonomes Parovirus, d.h. es benötigt zur Replikation kein Helfervirus.

Das Kapsid besteht aus zwei Polypeptiden mit Molekulargewichten von 83kDa (VP1) und 58kDa (VP2). Zusätzlich lassen sich drei Nicht-Strukturproteine von 52, 63 und 71kDa nachweisen.

Die DNA kodiert im 5'-Bereich für die Strukturproteine des Kapsids. Die kodierenden Bereiche dieser Strukturproteine sind bis auf einen zusätzlichen N-Terminus des VP1 identisch. Verursacht wird dieser Unterschied durch "splicing"-Vorgänge auf der mRNA-Ebene, bei denen im Fall von VP2 der translationelle Start für VP1 herausgenommen wird und somit die Translation erst mit dem kürzeren VP2 starten kann.

Untersuchungen an verschiedenen, weltweit gefundenen B19-Isolaten zeigten, daß sich diese zum Teil durch Restriktionsenzym-Muster auf DNA-Ebene unterscheiden. Diese Unterschiede korrelieren allerdings nicht mit dem klinischen Spektrum der B19-Infektion.

Bisher konnte keine permanente Zellinie gefunden werden, in der sich B19 vermehren läßt. Ebensowenig gelang es bisher, für B19 ein Versuchs-Tiermodell zu etablieren. Allerdings läßt sich B19 in primären Knochenmarks-Zellen unter Anwesenheit von Erythropoetin vermehren. So konnte der Replikations-Mechanismus des Virus geklärt und gezeigt werden, daß Zellen der Erythropoese Zielzellen dieser Infektion sind. Inzwischen gelang die Inokulation von B19 in fetalen erythropoetischen Zellen und Erythroblasten eines Patienten mit chronisch myeloischer Leukämie.

B19 verursacht das Erythema infectiosum (Ringelröteln), eine in der Regel benigne verlaufende Infektionserkrankung, die meist zwischen dem Kindes- und frühem Erwachsenenalter auftritt. Außerdem kann die B19-Infektion bei Patienten mit chronischhämolytischer Anämie (Sichelzellenanämie usw.) aplastische Krisen und bei Patienten mit angeborenen oder erworbenen Immunmangelzuständen chronische Knochenmarks-Aplasien verursachen.

In der Schwangerschaft kann die B19-Infektion in etwa 10 - 15% zu Hydrops fetalis mit resultierendem interuterinalem Fruchttod führen. Ferner ist B19 mit dem Auftreten der Purpura Schönlein-Henoch assoziert.

B19 wird in der Regel durch Tröpcheninfektion, aber auch durch antigen-positive Blutkonserven und Gerinnungspräparate übertragen.

Da bisher keine permanente Zellinie bekannt ist, in der sich B19 in großen Mengen gewinnen läßt, fehlt so eine Quelle zur Gewinnung von Antigen für diagnostische Tests. Bisher behilft man sich mit B19-Virus, das in Blutkonserven von Spendern, die sich gerade in virämischen Stadium der Infektion befinden, zufällig entdeckt wird.

Aufgabe der vorliegenden Erfindung ist es, immunologisch aktive Polypeptide zur Verfügung zu stellen, die mit den hier vorgestellten Testsystemen den Nachweis von B19-spezifischen Antikörpern der IgG und IgM-Klasse ermöglichen. Damit ergeben sich folgende Anwendungsmöglichkeiten:

- Serumdiagnose akuter bzw. abgelaufener B19-Infektionen in der Dermatologie, Hämatologie, Gynäkologie, Rheumatologie und Pädiatrie.
- Bestimmung des B19-Immunstatus bei Schwangeren
- Untersuchung von Blutkonserven oder Plasmaspenden zum Ausschluß der Übertragung von B19-Antigen, da durch anti-B19-IgG positives Blut oder Plasma höchstwahrscheinlich kein B19-Virus mehr übertragen werden kann.
- Selektion von anti-B19 positiven Plasmaspendern für die Produktion von B19-Hyperimmunglobulin-Präparaten.

Aufgrund des breiten klinischen Spektrums der durch B19 verursachten Erkrankungen sowie der Gefährdung für B19-seronegative Schwangere ist die Einführung von Testreagenzien dringend von Nöten.

Es hat sich herausgestellt, daß brauchbare immunologisch aktive Polypeptide nicht ohne weiteres hergestellt werden können. Die gentechnologische Darstellung von kurzen Peptiden ist ebenso wie die von großen Polypeptiden nur dann in einer zufriedenstellenden Ausbeute möglich, wenn geeignete Expressionsvektoren verwendet werden. Verhältnismäßig kurze Peptide können zwar leicht synthetisch hergestellt werden, erforderlich ist jedoch eine genauere Kenntnis der immunologischen Wirksamkeit.

Gegenstand der Erfindung sind immunologisch aktive Peptide, die einen Teil der Aminosäuresequenz der Kapsidproteine VP 1 oder VP 2 des Parvovirus B19 aufweisen. Diese Peptide sind dadurch gekennzeichnet, daß sie frei sind von Verunreinigungen, die den Nachweis von gegen Parvovirus B19 gerichteten Antikörpern stören. Diese Eigenschaft ist von großer Bedeutung, da solche Präparationen von Peptiden nicht brauchbar sind, die aufgrund der Herstellung Bestandteile enthalten, die mit den nachzuweisenden Antikörpern reagieren. Ein Beispiel für eine derart unerwünschte Verunreinigung ist Protein A, das spezifisch mit dem Fc-Anteil von IgG-Antikörpern reagieren kann. Ein besonderer Vorteil der erfindungsgemäßen immunologisch aktiven Peptide ist, daß sie durch die erfindungsgemäßen Herstellungsverfahren in guter Ausbeute hergestellt werden können. Werden nämlich die für einen diagnostischen Test benötigten Antigene nicht in einer ausreichenden Menge bei den Herstellungsverfahren synthetisiert, kann nicht die erforderliche Ausbeute nach den anschließenden Reinigungsverfahren erhalten werden.

Im Rahmen der vorliegenden Erfindung konnten weiterhin kurze Peptidsegmente aus dem VP 1, genauer aus dem Bereich von VP 1, der nicht mit VP 2 übereinstimmt, bestimmt werden, deren Epitope zum zuverlässigen Nachweis von Antikörpern gegen Parvovirus B19 in den Untersuchungsflüssigkeiten, insbesondere Seren geeignet sind. Dieser Bereich wird im folgenden als VP 1-VP 2 bezeichnet. Abb. 3 zeigt beispielhaft die Anordnung von einigen Peptiden (PAPEP 1-PAPEP 8) in dem Bereich (VP 1-VP 2). Auch wenn diese Peptide bevorzugt sind, können ebenso andere Peptide mit 8-50 Aminosäuren, bevorzugt 10 bis 32 Aminosäuren, aus dem Bereich VP 1-VP 2 eingesetzt werden. Dieser Bereich entspricht etwa dem Polypeptid PAN1, das in Abb. 2-1 dargestellt ist.

In einer bevorzugten Ausführungsform der vorliegenden Erfindung wird dieser kleine, immundominante und B19-spezifische Bereich im serologischen Test eingesetzt. Besonders bevorzugt wird dabei ein Gemisch synthetischer Peptide eingesetzt, wobei diese Peptide die in Beispiel 3 gezeigten Aminosäuresequenzen PAPEP 1 - PAPEP 8 aufweisen.

In einer anderen bevorzugten Ausführungsform der vorliegenden Erfindung werden die in Abbildung 2 dargestellten Aminosäuresequenzen der gentechnologisch dargestellten immunologisch aktiven Peptide PAN-1, PAN-2, PAN-3, PAN-4, PCE, PANSE UND PAPST eingesetzt. Hierbei ist es in der Regel ausreichend, wenn ein Peptid in dem Test verwendet wird. In besonderen Fällen können aber auch zwei oder mehr dieser Peptide eingesetzt werden.

Hergestellt werden können die erfindungsgemäßen Peptide entweder synthetisch oder gentechnologisch. Bevorzugt werden die kurzen Peptide, die in Beispiel 3 näher erläutert sind, synthetisch hergestellt. Die längeren Peptide jedoch werden bevorzugt gentechnologisch hergestellt.

Zunächst wurden die kodierenden Bereiche der viralen DNA mittels zweier Polymerase-Ketten-Reaktionen (polymerase chain reaction, PCR) aus dem Serum eines infizierten Patienten amplifiziert und in Plasmiden für die weitere Vermehrung in Escherichia (E.) coli kloniert. Nach weiteren Subklonierungsschritten wurden dann verschiedene Bereiche daraus in E. coli gentechnologisch exprimiert und die dabei entstehenden Antigene auf ihre Verwendung für den Nachweis von Antikörpern gegen das Virus untersucht. Eine direkte Herstellung der erfindungsgemäßen Peptide in Expressionsvektoren ist aufgrund verschiedener Schwierigkeiten nicht möglich. Erfindungsgemäß wird daher das virale Proteinsegment an ein stabil exprimierbares Protein anfusioniert. Dieses Fusionsprotein kann nach der Aufreinigung direkt als Antigen für den IgG-Nachweis eingesetzt werden. Bevorzugt wird jedoch der Parvovirus spezifische Anteil durch geeignete Methoden abgespalten, weiter aufgereinigt und dann für serologische Tests eingesetzt.

Gegenstand der vorliegenden Erfindung sind weiterhin Testkits für die Bestimmung von Antikörpern, die gegen Parvovirus B19 gerichtet sind. Die erfindungsgemäßen immunologisch aktiven Peptide können grundsätzlich in allen diagnostischen Testkits zum Nachweis von Antikörpern gegen Parvovirus B19 verwendet werden. In einer bevorzugten Ausführungsform der erfindungsgemäßen Testkits wird die Festphase geeigneter Mikrotiterplatten oder Polystyrol-Kugeln mit den erfindungsgemäßen immunologisch aktiven Peptiden beschichtet. Nach Inkubation mit der Untersuchungsflüssigkeit (Serum-Probe) in einer geeigneten Verdünnung wird nach üblichen Waschschritten Enzym- oder radioaktiv-markiertes anti-human-IgG zugegeben. Das Ausmaß der Substratumsetzung bzw. der gebundenen Radioaktivität zeigt dann an, ob in der Serum-Probe gegen Parvovirus B19 gerichtete Antikörper vorhanden sind.

Die erfindungsgemäßen Testkits werden üblicherweise an Laboratorien von Ärzten, Krankenhäusern, Untersuchungsanstalten etc. geliefert. Sie enthalten regelmäßig alle zur Testdurchführung benötigten Reagenzien. Übliche Testreagenzien wie Pufferlösungen etc. werden jedoch manchmal nicht mitgeliefert. In der Regel enthalten die Testkits Mikrotiterplatten oder Polystyrolkugeln, die entweder mit einem oder mehreren erfindungsgemäßen Peptiden, oder mit anti-Antikörpern beschichtet sind. Weiterhin können die Testkits, je nach Testprinzip, ein oder mehrere erfindungsgemäße Peptide enthalten. Schließlich umfassen die Testkits noch eine Anzeigekomponente, die eine Auswertung des Testergebnisses ermöglicht.

Bei anderen bevorzugten Testkits werden die Antigene an die Festphase von Mikrotiterplatten oder Polystyrol-Kugeln gebunden. Nach Inkubation des Testserums, geeigneten Wasch- und Absättigungsschritten wird ein spezifischer, enzymatisch oder radioaktiv markierter Antikörper gegen die B19-Antigene zugegeben und dessen Substratumsetzung bzw. die gebundene Radioaktivität gemessen. Da es sich hierbei um einen Inhibitionstest handelt, zeigt eine geringe Substratumsetzung bzw. geringe Radioaktivität das Vorhandensein von spezifischen Antikörpern an.

Für den Nachweis von IgM-Antiköpern gegen B19 können die an Festphasen gekoppelten erfindungsgemäßen Peptide ebenfalls eingesetzt werden. Bei diesem Nachweisverfahren werden zunächst die IgG-Antikörper durch Zugabe von mit Protein A beschichteten Kugeln zu den Untersuchungsflüssigkeiten eliminiert. Der Nachweis von gebundenen Antikörpern erfolgt dann mit einem anti-human IgM-Antikörper, der enzymatisch oder radioaktiv markiert ist.

Bei einem weiteren bevorzugten Testkit wird das Prinzip des sog. "µ-capture assays" verwendet. Mittels an die feste Phase gebundenen anti-human IgM-Antikörpern wird zuerst das IgM aus der Untersuchungsflüssigkeit (Serum) gebunden. Dann werden die erfindungsgemäßen immunologisch aktiven Peptide zugegeben. Das Ausmaß der Bindung der Antigene und somit die Menge an vorhandenem Anti-B19-IgM kann dadurch erfolgen, das entweder die Antigene radioaktiv oder mit anderen Substanzen (Digoxigenin, Avidin) markiert sind und somit nachweisbar sind, oder daß ein zweiter markierter Antikörper gegen die B19-Antigene eingesetzt und dessen Bindung gemessen wird.

Im Rahmen der vorliegenden Erfindung ganz besonders bevorzugt sind die ELISA-(enzyme linked immunosorbent assay)-Testkits.

Erfindungsgemäß werden auch DNA-Sequenzen zur Verfügung gestellt, die für den direkten Nachweis des Virus in Untersuchungsproben (Seren, Biopsien etc) verwendet werden können. Bevorzugt werden zwei DNA-Primer verwendet, die sich spezifisch an DNA-Bereiche im VP 1 anlagern. Mittels eines käuflich erhältlichen Polymerase-chain-reaction-Kits kann dann eine Amplifizierung des dazwischen liegenden Bereiches erreicht werden. Nachgewiesen wird die dann in geeigneter Weise fixierte amplifizierte DNA durch eine geeignete DNA-Sequenz. Hergestellt wird diese für die Hybridisierung eingesetzte DNA mit Hilfe eines Plasmides, das den zwischen den beiden Primern liegenden DNA-Bereich enthält.

Selbstverständlich dürfen die Primer Sequenzen nicht in der zur Hybridisierung eingesetzten DNA vorhanden sein. Die Sequenz der verwendeten Primer und deren Anordnung zueinander ist in Abb. 1 dargestellt.

Schließlich werden im Rahmen der vorliegenden Erfindung auch Impfstoffe gegen Parvovirus B19 zur Verfügung gestellt. Hierbei werden die erfindungsgemäßen immunologisch aktiven Peptide zusammen mit geeigneten Adjuvantien den zu schützenden Personen gegebenenfalls mehrmals appliziert. Die dadurch hervorgerufene Produktion von Antikörpern kann einen Schutz vor Infektion mit Parvovirus B19 bewirken.

BEISPIEL 1:

Gewinnung von Parvovirus B19 VP 1- und VP 2-kodierenden Sequenzen aus Patientenserum

Von einem Patienten mit akuter Infektion (Erythema infectiosum) wurde aus 1ml Serum virale DNA durch Proteinase K-Verdau in 1% SDS, Phenolextraktion und anschließender Alkohol-Fällung isoliert (Diese und auch alle folgenden Schritte zur Gewinnung, Verarbeitung und Expression von DNA, sowie auch die Darstellung von rekombinanten Proteinen und grundlegende Schritte zu deren Reinigung sind in: Maniatis, T., Fritsch, E.F., Sambrook, J. (1982) Molecular cloning. Cold Spring Harbor, N.Y. ausführlich dargestellt). Diese DNA wurde in 50µl TE-Puffer aufgenommen und jeweils dann 1µl für die Amplifizierung mittels der "polymerase chain reaction" und synthetischen Oligodesoxynukleotiden eingesetzt. Für die Amplifizierung der kodierenden Bereiche der Oberflächenproteine wurden zwei Primer-Paare verwendet; eines davon für die Gewinnung des VP 1-Anteils, das zweite Paar für das gesamte VP 2. Die als Primer verwendeten Oligodesoxynukleotide besitzen an ihren jeweiligen 5'-Enden Sequenzen, die nicht mit der Parvovirus-Sequenz homolog sind, für Restriktionsenzym-Schnittstellen kodieren und deswegen geeignet sind, die aus der PCR entstandenen DNA-Fragmente in geeignete Vektoren zu klonieren. Es wurden die in Abb. 1 mit 0-1 bis 0-5 bezeichneten Primer verwendet.

Jeweils fünf Ansätze mit je 1µl isolierter Parvovirus DNA wurden mit den beiden Primer-Paaren in einem Volumen von 100µl amplifiziert. Die Bedingungen dabei waren: 1,5min Denaturierung bei 94°C, 2min Anlagerung der Primer bei 45°C, 4min Synthese bei 72°C; insgesamt 50 Zyklen; Puffer, Substrate und Taq-Polymerase wurden dabei nach Angaben des Herstellers (Cetus/Perkin-Elmer, Überlingen, BRD) eingesetzt.

Die amplifizierten DNA-Fragmente aus den beiden unterschiedlichen Ansätzen (für VP 1 und VP 2) wurden jeweils vereinigt, mittels Alkohol-Fällung präzipitiert, mit 70% Alkohol gewaschen, getrocknet, in einem Volumen von 200µl TE-Puffer gelöst und mit den Restriktionsenzymen EcoRI und HindIII verdaut. Nach elektrophoretischer Auftrennung der Fragmente in einem 1,2%igen Agarose-Gel erfolgte dann die Isolierung der entsprechenden DNA-Banden (709bp für VP 1, 1704bp für VP 2) und Insertion in die EcoRI und HindIII Stellen des Vektors PUC12 (Pharmacia, Schweden). Nach Transformation der Plasmide in E.coli JM109 (Pharmacia, Schweden) wurden Bakterienklone mit Parvovirus-DNA-Inserts durch Restriktionsverdau charakterisiert. Die entsprechenden Klone erhielten die Namen pUC12PAN für den VP 1-Anteil-kodierenden Bereich und pUC12VP2 für den VP 2-kodierenden Bereich.

BEISPIEL 2

Gentechnologische Darstellung von VP 1-Anteil und VP 2 aus E.coli-Zellen

a) VP1-Anteil:

1) PAN-1

Aus dem Plasmid pUC12PAN wurde der VP1-kodierende Bereich mit BclI und HindIII (siehe Abb.1, die HindIII-Stelle stammt aus dem pUC-Vektor) isoliert und hinter das 3'-Ende eines verkürzten β-Galactosidase-Gens des Vektors (z.B. pBD2) in die Restriktionsschnitt-Stellen BamHI und HindIII inseriert. E.coli-Zellen mit daraus resultierenden Plasmiden exprimieren nach Induktion mit IPTG in großer Menge ein B-Gal::VP1-Fusionsprotein (ca. 67kDA), welches im Immunoblot (Western Blot) sehr stark mit anti-Parvovirus B19-positiven Seren reagiert. Eine Reinigung dieses Proteins kann sehr leicht mit konventionellen Methoden erreicht werden, indem die Unlöslichkeit des Proteins ausgenutzt wird. Nach Lyse der Zellen wird die Pelletfraktion mit Detergentien wie Triton-X100 und Octyl-gluco-pyranosid gewaschen, das Fusionsprotein anschließend mit 8M Harnstoff/1% Mercaptoethanol in Lösung gebracht und durch DEAE-Chromatographie mit einem NaCl-Gradienten von zellulären Verunreinigungen abgetrennt.

Eine Abspaltung des VP1-Anteils aus dem Fusionsprotein kann durch BrCN-Spaltung erreicht werden, da die VP1-Proteinsequenz mit einem Methionin beginnt und im Fragment selbst diese Aminosäure nicht mehr auftaucht; im bakteriellen Fusionsanteil dagegen tritt Methionin relativ oft auf, so daß dieser Anteil in sehr kleine Bruchstücke zerlegt wird. Nach der Spaltung in 35% Ameisensäure und 0,1mg/ml BrCN für 4h bei Raumtemperatur wurde die Probe lyophilisiert, in 8M Harnstoff, 2mM DTE (Dithiothreitol) gelöst und mittels DEAE-Chromatographie in einem NaCl-Gradienten aufgereinigt. Das hieraus resultierende VP1-Fragment wurde PAN-1 benannt und kann direkt für serologische Bestimmungen verwendet werden. Die Aminosäure-Sequenz ist in Abb.2-1 angegeben.

Als weitere Konstrukte wurden Plasmide generiert, die für Fusionsproteine bestehend aus der Glutathion-S-Transferase aus Schistosoma japonicum (Smith, D.B. and Johnson, K.S.:Single step purification of polypeptides expressed in Escherichia coli as fusions with glutathione S-transferase. Gene, 67 (1988) 31-40) und dem VP1-Anteil kodieren. Es kann aber auch ein anderer Fusionspartner verwendet werden, solange dieser nicht den diagnostischen Test stört.

2) PCE:

Aus pUC12PAN wurde das B19-DNA-Fragment nach BclI/PvuII Verdau (618bp) isoliert und in pGEX1 in die BamHI und SmaI Stellen integriert (pGEXIPAN). Das entstehende 52kDa-Fusionsprotein wurde mittels Glutathion-gekoppelter Agarose aus dem Überstand gereinigt und als Antigen für die serologischen Teste in Beispiel 4 eingesetzt (Name:PCE). Die Aminosäure-Abfolge dieses Antigens ist in Abb. 2-2 aufgeführt.

3) PAN-2:

Aus pUC12PAN wurde ein 458bp Fragment mit BclI/HincII isoliert und nach Zwischenklonierungen in anderen Vektoren in pGEX2 (pGEX2PAN) inseriert. Die Insertion des Fragments im selben Leserahmen kann auch durch die Verwendung von synthetischen Oligodesoxynukleotiden erreicht werden. An der Fusions-Stelle von Glutathion-S-Transferase und dem VP1-Segment ist die Aminosäure-Sequenz, die von Thrombin erkannt wird, so daß der B19-Anteil durch dieses Enzym vom Fusionspartner abgespalten werden kann. Es kann auch jeder andere Fusionspartner verwendet werden, wenn er diese Protease-Erkennungssequenz besitzt. Die Aminosäure-Sequenz des Antigens, sowie anfusionierte Fremdaminosäuren (in Fettdruck) ist in Abb. 2-3 angegeben.

4) PAN-3:

Aus pUC12PAN wurde ein 458bp Fragment mit BclI/HincII isoliert und nach Zwischenklonierungen in anderen Vektoren in pGEX3 (pGEX3PAN) inseriert. Die Insertion des Fragments im selben Leserahmen kann auch durch die Verwendung von synthetischen Oligodesoxynukleotiden erreicht werden. An der Fusions-Stelle von Glutathion-S-Transferase und dem VP1-Segment ist die Aminosäure-Sequenz, die vom Protease Faktor Xa erkannt wird, so daß der B19-Anteil durch dieses Enzym vom Fusionspartner abgespalten werden kann. Es kann auch jeder andere Fusionspartner verwendet werden, wenn er diese Protease-Erkennungssequenz besitzt. Die Aminosäure-Sequenz des Antigens, sowie anfusionierte Fremdaminosäuren (in Fettdruck) ist in Abb. 2-4 angegeben.

5) PAN-4:

Aus pUC12PAN wurde das gesamte B19-DNA-Insert durch BclI und PstI Verdau gewonnen und nach verschiedenen ZwischenklonierungsSchritten in den Vektor pGEX2 inseriert. Erhalten wurde dabei das Plasmid pGEX2PAN. Die Insertion des Fragments im selben Leserahmen kann auch durch die Verwendung von synthetischen Oligodesoxynukleotiden erreicht werden. An der Fusions-Stelle von Glutathion-S-Transferase und dem VP1-Segment ist die Aminosäure-Sequenz für die Protease Thrombin, so daß der B19-Anteil durch dieses Enzym vom Fusionspartner abgespalten werden kann. Es kann auch ein geeigneter anderer Fusionspartner verwendet werden, wenn er diese Protease-Erkennungssequenz besitzt. Die Aminosäure-Sequenz des Antigens, sowie anfusionierte Fremdaminosäuren (in Fettdruck) ist in Abb. 2-5 angegeben.

Eine Reinigung der Antigene kann durch einfache Affinitäts-Chromatographie mit einer Glutathion-gekoppelten Gelmatrix erreicht werden.

Zur weiteren Aufreinigung der auf pGEX 2 und 3 beruhenden Fusionsproteine wurde eine Abspaltung des B19-Anteils durch Verdau mit Trombin bzw Faktor X nach Angaben des Herstellers (Boehringer Mannheim) durchgeführt. Die Bruchstücke wurden dann nochmals Affinitäts-chromatographisch aufgereinigt. Die S-Glutathion-Transferase kann dabei selektiv herausgefischt werden, der Parvovirus Proteinanteil ist in Durchlauf zu finden und kann nach einer abschließenden DEAE-chromatographischen Auftrennung in serologischen Tests eingesetzt werden.

Alternativ hierzu kann aber auch die Protease direkt zu der Glutathion-gekoppelten Gelsuspension mit dem angebundenen Fusionsprotein gegeben werden. Nach einer Inkubationszeit von ca. 1h läßt sich das abgespaltene VPI-Fragment aus dem Gel waschen, während der Glutathion-S-Transferase-Anteil an die Gelmatrix gebunden bleibt.

b) VP-2-Anteil:

1) VP-2

Bedingt durch die Auswahl der PCR-Primer sowie des Vektors ist der kodierende Bereich für VP2 im Plasmid pUC12VP2 bereits im richtigen Leserahmen und kann nach Induktion mit IPTG aus der unlöslichen Fraktion des Bakterienlysats, ähnlich wie für pBD2PAN beschrieben, aufgereinigt werden. Die Aminosäure-Sequenz des rekombinanten Antigens ist in Abb.2-6 gezeigt.

2) PANSE:

Überraschenderweise zeigte sich, daß eine Verkürzung der VP2-kodierenden Sequenz eine erhebliche Steigerung der Proteinausbeute mit sich bringt, daß dieses verkürzte Antigen stabil exprimierbar ist, auch während der Reinigung nicht degradiert wird sowie auch noch dieselbe Rektivität mit anti-B19 positiven Seren aufweist. Dieses Expressionsplasmid (pUC19PANSE) wurde erhalten, indem der 5'-Bereich von VP2 um 355bp bis zu einer NsiI-Stelle verkürzt wurde. Dieses Fragment wurde in pUC19 (Pharmacia, Schweden) inseriert, das denselben Leserahmen im lacZ-Peptid aufweist wie die B19 Sequenz. Da aufgrund der PCR Primer am 3'-Ende eine HindIII-Stelle sitzt, mußte durch Zwischenklonierung noch eine EcoRI-Stelle geschaffen werden, um das gewünschte Fragment in die PstI und EcoRI Stellen von pUC19 inserieren zu können.

Das Antigen mit einer Größe von ca. 38kDa (PANSE) kann sehr einfach aus der Pellet-Fraktion des Bakterien-Lysats nach Lösen in 4M Harnstoff durch DEAE-Chromatographie von Verunreinigungen getrennt werden. Die Aminosäure-Sequenz des Antigens ist in Abb. 2-7 angegeben.

3) PAPST:

Von dem Plasmid pUC12VP2 wurde durch PstI Verdau ein 716bp großes Fragment isoliert, das den N-terminalen Bereich von VP-2 kodiert. Nach Insertion des Fragments in den Vektor pUC9 (Pharmacia, Schweden) in die selbe Orientierung der Leserahmen wie dem lacZ des Vektors (charakterisiert durch Restriktionsenzym-Verdau) wird das B19-Antigen mit einer Größe von ca 33kDa in sehr großer Menge (ca. 10% des Gesamt-E.coli-Proteins) produziert. Eine Aufreinigung kann ähnlich wie bei pBDAN aus den unlöslichen Bestandteilen durch Lösen in 8M Harnstoff und anschließende DEAE-Chromatographie erfolgen. Die Aminosäure-Sequenz ist in Abb.2-8 aufgezeigt.

c) gesamtes VP1/VP2:

Das Plasmid pUC12PAN wurde mit PstI und HindIII geöffnet und der VP2 kodierende Bereich aus pUC12VP2 nach HindIII und partiellem PstI-Verdau als 1,7kb Fragment inseriert (pUC12VP1/2).

6

Expression VP1/2-enthaltender Antigene in E. coli:
1) PAV-1-B:

pUC12VP1/2 wurde mit EcoRI und BamHI geschnitten, eine 1466 bp große DNA-Bande isoliert und dieses anschließend in die EcoRI/BamHI Stellen des Vektors pUC18stop eingesetzt. pUC18 stop gleicht den oben erwähnten pUC-Vektoren; im Unterschied zu diesen enthält er jedoch zwischen der PstI und HindIII Stelle ein Synthetisches Oligodesoxynukleotid, das für Translations-Stop-Signale sowie für den Stop der Transkription kodiert. Somit hat die Polylinker-Region des Vektor folgende Sequenz:

```
ATG ACC ATG ATT ACG AAT TCG AGC TCG GTA CCC GGG GAT CCT CTA GAG
TCG ACC TGC AGT AAT TAA TTA GAT CTC GAG CCC GCC TAA TGA GCG GGC
TTT TTT AAG CTT
```

(Die Restriktionsschnittstellen EcoRI - GAATTC, BamHI - GGATCC, PstI - CTGCAG, BglII - AGATCT und HindIII - AACGTT sind durch Fettdruck markiert)

Der so erhaltene Vektor (pUC-V1-B) kodiert das VP-1-Strukturprotein vom Start bis zur Aminosäure 486 gefolgt von einigen Aminosäuren des pUC-Polylinkers und wird terminiert durch die Stop-Codons des inserierten Oligodesoxynukleotids. Das exprimierte Antigen (PAV-1-B) wird in sehr guter Ausbeute nach IPTG-Induktion in den E.coli-Zellen produziert und besitzt eine Größe von 60 kDa. Seine Aminosäuren-Abfolge ist in Abb. 2-9 dargestellt, durch Fettdruck hervorgehobene Aminosäuren sind nicht B19-spezifisch und werden durch pUC-Sequenzen kodiert. Die Reaktivität mit anit-B19-positiven Seren ist sehr gut und eine effiziente Aufreinigung läßt sich durch Entfernen löslicher E.coli-Proteine, Lösen in 8M Harnstoff und konventioneller Ionen-Austauscher-Chromatographie (wie beschrieben) erreichen.
2) PAV-1-N:

Der oben beschriebene Vektor pUCVP-1-B wurde mit EcoRI und NsiI verdaut. Die so entstehende 1137bp große Bande wurde in den Vektor pUC18stop in die EcoRI und PstI Stellen inseriert (siehe oben). Der entstehende Vektor pUCVP-1-N kodiert das Strukturprotein vom Start bis zur Aminosäure 377; das Antigen (PAV-1-N) wird nach IPTG-Induktion in den E.coli-Zellen etwas schlechter produziert als das oben beschriebene Antigen PAV-1-B. Seine Größe ist 45 kDa, die Reaktivität mit anti-B19 Seren gut. Die Aminosäurenabfolge ist in Abb. 2-10 angegeben, Aminosäuren mit Fettdruck werden vom pUC-Vektor kodiert und sind nicht B19 spezifisch.

Die Aufreinigung des Antigens kann wie bei PAV-1-B erreicht werden.
3) Expression der unter c)1) und c)2) beschriebenen Antigene als GST-Fusionsproteine

Die beiden Vektoren pUCVP-1-B und pUCVP-1-N wurden mit EcoRI/BglII verdaut und die entstehenden ca. 1480bp bzw. 1150bp großen Banden isoliert, wobei die vom pUC18stop mit eingeführten Translations-Stop-Signale mit übernommen werden. (Die BglII-Stelle ist in der oben angeführten pUC-18stop-Polylinker-Sequenz angegeben, die BclI-Stelle (TGATCA) unmittelbar vor dem Start der Translation wurde mit den für die DNA-Amplifikation verwendeten Primern eingeführt - siehe Abb. 1, 0-1). Die beiden Fragmente, die dieselben 5'-Sequenzen, jedoch unterschiedlich lange Bereiche von VP-1 kodieren, wurden nun in den oben beschriebenen Vektor pGEX-1 eingesetzt. Da pGEX-1 für die Insertion des 3'-Endes eines Fragments nur die Restriktions-Schnitt-Stellen SmaI und EcoRI zur Verfügung hat, mußte zuerst noch eine für SmaI (blunt end) kompatible Stelle geschaffen werden. Dies erfolgte durch Insertion der beiden DNA-Fragmente in die Restriktions-Stellen EcoRI und BamHI (kompatibel mit BglII) des Vektors pIC20H (The pIC plasmid and phage vectors with versatile cloning sites for recombinant selection by insertional inactivation, J.L. Marsh, M. Erfle and E.J. Wykes, Gene, 32 (1984) 481-485). Von den beiden entstandenen Vektoren wurden nun die beiden Fragmente mit BclI und HincII (blunt end - Schnitt) wiederum isoliert und in pGEX-1 in BamHI und SmaI inseriert. Da HincII auch in der B19-Sequenz schneidet, wurden die beiden Fragmente durch einen partiellen HincII-Verdau isoliert.

Die beiden pGEX-Vektoren exprimieren nun Fusionsproteine bestehend aus der Glutathion-S-Transferase gefolgt von den beiden unterschiedlich langen VP-1 Segmenten. Das aus pUCVP-1-B stammende und nun in pGEXVP-1-B sitzende Fragment ergibt ein Fusionsprotein mit ca. 87kDa; das kleinere, nur bis Aminosäure 377 kodierende Fragment ein Fusionsprotein in der Größe von 72kDa.

Die Aminosäureabfolgen sind in Abb. 2-9 und 2-10 angegeben. Der einzige Unterschied ist, daß die fünf N-terminalen Aminosäuren wegfallen und statt dessen durch die Glutathion-S-Transferase ersetzt sind.
4) Weitere Expression von VP1/VP2:

Aus dem Plasmid pUC12VP1/2 mit der gesamten VP1 und VP2 kodierenden Region wurde nach EcoRI- und HindIII-Verdau ein 2,4kb Fragment isoliert und in den eukaryotischen Expressionsvektor pMDIII (Motz, M., Deby, G., Jilg, W., Wolf, H.: Expression of the Epstein-barr virus major membrane proteins in Chinese hamster ovary cells. Gene, 44 (1986) 353-359. (Erhältlich bei ATCC)) nach EcoRI und HindIII Verdau inseriert. Dieses Plasmid wurde anschließend wieder mit SalI linearisiert und noch ein 2,4kb SalI-Fragment mit einem Dihydrofolat-Reduktase-Gen (DHFR) sowie Regulationssequenzen eingesetzt. Das so erhaltene Plasmid pMDIIIVP1/2 wurde in DHFR-negative CHO-Zellen transfiziert. Nach Selektion auf alpha-minus Medium (gibco) wurden entstehende Kolonien isoliert und nach dem Auswachsen mit steigenden Konzentrationen an Methotrexat (MTX) amplifiziert. Von diesen Zellkulturen können Partikel mit VP1/VP2 aus dem Kulturüberstand gereinigt werden.

Weiterhin wurde das 2,4kb-Fragment aus pUC12VP1/2 nach EcoRI/HindIII-Verdau in einen Vektor inseriert, der neben der HindIII-Stelle noch eine BamHI-Stelle besitzt. Von diesem Zwischenkonstrukt wurde dann der Parvovirus-Anteil als BclI und BamHI-Fragment isoliert und in die BamHI-Stelle eines Bakulovirus-Expressionsvektors (verwendet werden können verschiedene Konstrukte) inseriert. (Die BclI-Stelle sitzt unmittelbar vor dem translationellen Start von VP1, sie ist durch die PCR-Primer kodiert, siehe Abb. 1; der BamHI-Verdau muß partiell durchgeführt werden, da in der Parvovirus-Sequenz sich auch noch eine solche Stelle befindet.). Nach co-Transfektion des entstandenen Plasmids mit Wildtyp-Bakulovirus-DNA in eine Insekten-Zellkultur-Linie werden Zellen isoliert, die keine sogenannten "inclusion bodies" aufweisen. Aus solchen Zellen, bei denen das Bakulovirus Polyhedrin-Gen durch das VP1/2-Gen ersetzt ist, läßt sich das intrazellulär gebildete VP1 in großer Menge aufreinigen.

5) Expression von VP-2:

Weiterhin wurde die Expression des kleineren B19-VP-2 mittels rekombinanter Bakuloviren erhalten. Hierfür wurde das VP-2-kodierende Plasmid pUC12VP (siehe Beispiel 1) mit EcoRI und HindIII verdaut und das resultierende 1,7kb Fragment in einen oben erwähnten Vektor inseriert, der neben der HindIII-Stelle noch eine BamHI-Stelle besitzt. Von diesem Zwischenkonstrukt wurde dann der Parvovirus-Anteil als BclI und BamHI-Fragment isoliert und in die BamHI-Stelle eines Bakulovirus-Expressionsvektors (verwendet werden können verschiedene Konstrukte) inseriert. (Die BclI-Stelle sitzt unmittelbar vor dem translationellen Start von VP2, sie ist durch die PCR-Primer kodiert, siehe Abb. 1, 0-3; der BamHI-Verdau muß partiell durchgeführt wreden, da in der Parvovirus-Sequenz sich auch noch eine solche Stelle befindet). Nach co-Transfektion des entstandenen Plasmids mit Wildtyp-Bakulovirus-DNA in eine Insekten-Zellkultur-Linie werden Zellen isoliert, die keine sogenannten "inclusion bodies" aufweisen. Aus solchen Zellen, bei denen das Bakulovirus Polyhedrin-Gen durch das VP2-Gen ersetzt ist, läßt sich das VP2 in großer Menge als Partikel aufreinigen. Diese Partikel sind besonders für den Einsatz im $\mu$-capture-Test geeignet.

BEISPIEL 3

Synthetische Peptide mit immundominanten Epitopen

Aus den Reaktionsmustern der bakteriellen Expressionsprodukte (insbesondere pGEX::VP1-Fusionskonstrukte) mit Parvovirus-positiven Seren im Western Blot läßt sich die überraschende Folgerung ziehen, daß ein kurzes Fragment aus dem VP1 Anteil ausreichend ist, um alle IgG-positiven Parvoseren zu identifizieren.

Das Fragment läßt sich mit folgenden synthetisch produzierbaren Peptiden abdecken:

PAPEP-1:

```
Ser Lys Lys Ser Gly Lys Trp Trp Glu Ser Asp Asp Lys Phe Ala Lys
Ala Val Tyr
```

PAPEP-2:

```
Leu Lys Asp His Tyr Asn Ile Ser Leu Asp Asn Pro Leu Glu Asn Pro
Ser Ser
```

PAPEP-3:

```
Ile Lys Asn Asn Leu Lys Asn Ser Pro Asp Leu Tyr Ser His His Phe
Gln Ser His Gly Gln Leu Ser Asp His Pro His Ala
```

PAPEP-4:

```
Ser Ser His Ala Glu Pro Arg Gly Glu Asn Ala Val Leu Ser Ser Glu
Asp Leu His Lys Pro Gly Gln Val
```

PAPEP-5:

```
Asn Tyr Val Gly Pro Gly Asn Glu Leu Gln Ala Gly Pro Pro Gln Ser
Ala Val Asp Ser Ala Ala Arg Ile His Asp Phe Arg Tyr Ser Gln Leu
```

PAPEP-6:

```
Pro Tyr Thr His Trp Thr Val Ala Asp Glu Glu Leu Leu Lys Asn Ile
Lys Asn Glu Thr Gly Phe
```

PAPEP-7:

```
Asn Ala Ser Glu Lys Tyr Pro Ser Met Thr Ser Val Asn Ser Ala Glu
Ala Ser
```

PAPEP-8:

```
Asn Pro Tyr Thr His Trp Thr Val Ala Asp Glu Glu Leu Leu Lys His
Ile Lys
```

Diese Antigene können in großen Mengen synthetisch problemlos hergestellt, aufgereinigt und dann im ELISA in Konzentrationen zwischen 100 - 200 ng pro Ansatz eingesetzt werden.

Auch wenn bereits mit einem der Peptide gute Ergebnisse ereicht werden können, ist die gemeinsame Verwendung von zwei oder drei Peptiden bevorzugt.

Für den Nachweis von IgG- oder IgM-Antikörpern können zum Teil unterschiedliche Peptide oder Kombinationen davon Verwendung finden.

BEISPIEL 4

a) Bestimmung von Serumantikörpern gegen das Parvovirus B19

Mit den in Beispiel 2 beschriebenen und gereinigten Antigenen wurde eine größere Menge an Seren auf ihre Reaktivität mit diesen Antigenen getestet. Dabei wurden die verschiedenen rekombinanten Proteine in einer Konzentration von 0,5 - 1 µg/ml in Carbonatpuffer, pH 9,5, in die Näpfe kommerziell erhältlicher ELISA-Platten für 16h zum Anbinden gegeben. Nach dem Abwaschen von nicht-gebundenem Material können diese Platten dann im getrockneten Zustand bei 4°C gelagert werden.

Die Inkubation mit den Seren für 2h erfolgte in einer Verdünnung von 1:100, die anschließenden Waschprozeduren und der Nachweis gebundener Antikörper mit einem Peroxidase-gekoppelten anti-human-IgG Antikörper erfolgte nach üblichen Testprozeduren.

Getestet wurden verschiedene Serumpanels auf anti-B19-IgG:

1. Seren eines Patienten mit akuter B19-Infektion wurden konsekutiv ab Auftreten des Erythema infektiosum bis 19 Wochen nach der Erkrankung untersucht.

Ergebnis:

Sowohl mit dem Fusionsprotein aus pGEX1PAN (PCE, siehe Beispiel 2) und mit einem durch BrCN abgespaltenen VP-1 Bereich (PAN-1, siehe Beispiel 2) und auch mit einem durch Thrombin abgespaltenen VP1-Anteil (PAN-4) als Antigene wurden alle Seren bereits ab Beginn der klinischen Manifestation als anti-B19-IgG positiv erkannt und blieben über den Beobachtungszeitraum (19 Wochen) positiv.

2. Auf anti-B19-IgG wurden Serumpaare von Schwangeren (n=21) getestet, denen bei Hospitalisierung und vier Wochen später eine Serumprobe entnommen wurde. Es wurden für jedes Antigen dieselben Testseren verwendet.

Ergebnis:

PCE:

Von den 21 Schwangeren waren 15 zum Zeitpunkt der Hospitalisierung anti-B19 negativ, 6 Frauen anti-B19-IgG positiv. Das serologische Ergebnis der Zweitserumprobe vier Wochen später ergab ein identisches Ergebnis.

PAN-2:

Von den 21 Schwangeren waren 14 zum Zeitpunkt der Hospitalisierung anti-B19-IgG negativ, 7 Frauen anti-B19 positiv. Bei der Retestung von Serumproben, die vier Wochen später diesen Frauen abgenommen wurden, ließen sich bei einer Frau, die zuvor anti-B19-IgG positiv war, kein IgG mehr nachweisen.

PAN-4:

Von den 21 Schwangeren waren 15 zum Zeitpunkt der Hospitalisierung anti-B19 negativ, 6 Frauen anti-B19-IgG positiv. Das serologische Ergebnis der Zweitserumprobe vier Wochen später ergab ein identisches Ergebnis.

b) Austestung einer definiert B19 IgG/M-positiv/negativen Serum-Sammlung (n=13)

Die verwendeten Seren stammten von klinisch definierten Fällen und waren zuvor in einem IgG/M-Test überprüft worden, der gereinigtes Virus als Antigen verwendet. Seren 1-6: anti-B19 negativ, 7-9: IgM/IgG-positiv, 10-13; IgM-negativ, IgG-positiv.

Getestet wurden PAN-4 nach der oben beschriebenen Prozedur. Die Bestimmung der IgM-Antikörper erfolgte nach demselben Testprinzip wie bei der IgG-Bestimmung, jedoch wurden als Antigene PANSE und PAV-1-B in einer 1:1 Mischung mit einer 10-fach höheren Konzentration an die Platten gebunden, weiterhin erfolgte eine Elimination der Serum IgG-Antikörper durch Prä-Adsorption mit Protein A-gekoppelten Kugeln.

Folgende Absorptionswerte wurden erhalten:

IgG-Bestimmung mit PAN-4 (ca. 20ng pro Testnapf), IgM mit einer 1:1 Mischung aus PANSE und PAV-1-B (ca. 150ng pro Testnapf Gesamtprotein)

| Serum | IgG | IgM |
|---|---|---|
| 1 | 0,09 | 0,07 |
| 2 | 0,05 | 0,06 |
| 3 | 0,10 | 0,08 |
| 4 | 0,07 | 0,06 |
| 5 | 0,07 | 0,08 |
| 6 | 0,04 | 0,07 |
| 7 | 1,82 | 1,53 |
| 8 | 0,90 | 0,46 |
| 9 | 0,72 | 0,56 |
| 10 | 1,10 | 0,08 |
| 11 | 0,62 | 0,14 |
| 12 | 0,98 | 0,11 |
| 13 | 0,87 | 0,09 |

Die Ergebnisse zeigen eine klare Diskrimination der positiven/negativen Seren sowohl für den IgG- als auch für den IgM-Test.

Die verwendeten IgM-positiven Seren stammten von klinisch definierten Fällen und waren zuvor in einem IgM-Test überprüft worden, der gereinigtes Virus als Antigen verwendet. Ein Testansatz mit rekombinanten Antigenen aus dem VP1 und VP2 Bereich erkannte auch alle IgM-positiven Seren. Es zeigte sich, daß die VP2-Anteile "PAPST, VP2 aber besonders PANSE" hier besser reaktiv als im IgG-Test sind. In einem kommerziellen Testkit für IgM werden deshalb beide Bereiche vertreten sein.

Eine weitere Verbesserung der Sensitivität läßt sich durch selektives Anbinden der Serum-IgM-Antikörper an die feste Phase mittels monoklonaler Antikörper, Zugabe von rekombinantem Antigen (Baculovirusexprimiertes, partikuläres VP-2) sowie der Bestimmung der Anbindung erzielen (μ-capture assay).

Diese Versuche belegen die hohe Zuverlässigkeit des unter Verwendung der erfindungsgemäßen immunologisch aktiven Polypeptide durchgeführten Testes.

Der VP2-Bereich, enthalten in dem PANSE, PAPST und VP-2" benannten Antigenen bringt für die Bestimmung von Antikörpern aus Patienten mit lang abgelaufener Infektion keine zusätzliche Steigerung der Sensitivität. Eine gute Reaktion mit diesen Antigenen dagegen ist bei Seren mit erst kurz zurückliegender Infektion zu finden. Deshalb ist dieses Antigen geeignet, eine Aussage über den Zeitpunkt der Infektion zu machen.

In einem Testkit kann eines oder eine Mischung dieser Antigene entweder zu den gentechnologisch erzeugten VP1-Anteilen bzw. zum synthetischen Peptid zugemischt werden, oder aber in getrennten Ansätzen verwendet werden, wo die Diskriminierug der Reaktivität gegen diese beiden Bereiche eine zusätzliche Aussage über den Infektionszeitpunkt erlaubt.

Eine weitere Verbesserung der Sensitivität läßt sich durch ein selektives Anbinden der Serum-IgM-Antikörper an die feste Phase mittels monoklonaler Antikörper, Zugabe von rekombinantem Antigen sowie der Bestimmung der Anbindung erzielen (μ-capture assay).

Beispiel 5

Verwendung B19-spezifischer DNA-Primer für den direkten Erregernachweis

Eventuell vorhandene B19-DNA wurden aus den Untersuchungsproben (Serum, Biopsien) durch Proteinase K-Verdau in Gegenwart von 1% SDS (2h, 37°C), Phenol-Extraktion und Fällung in 70% Ethanol gewonnen. Diese und auch die dann folgende DNA-Amplifizierung wurde analog zu der in Beispiel 1 beschriebenen Prozedur durchgeführt. Verwendet wurden Primer 0-5 und 0-2 (Sequenz und Lage auf dem B19-Genom siehe Abb. 1); bei B19-positiven Proben besitzt das amplifizierte Fragment eine Größe von 319bp. Der Nachweis der B19-Spezifität des DNA-Fragments erfolgte nach Auftrennung der PCR-Ansätze durch ein 1,5%iges Agarose-Gel, Transfer der DNA auf eine Nitrozellulose-Membran (Southern Blot) und Hybridisierung mit einem dazwischen-liegenden DNA-Stück, das entweder radioaktiv mit 32P oder Digoxygenin mittels konventioneller Methoden (primer extension) markiert worden war. Das zur Hybridisierung verwendete DNA-Fragment wurde auf folgende Art gewonnen: Aus dem Plasmid pUC12PAN wurde nach Verdau mit HincII und PstI ein 260bp langes DNA-Fragment isoliert und in pUC12 in die HincII und PstI Stellen inseriert. Aus dem resultierenden Plasmid (pUC12PCRDIA) kann nun das B19-Fragment ohne die zur Amplifizierung verwendeten Sequenzen durch EcoRI/PstI-Verdau gewonnen werden und nach Markierung als Hybridisierungsprobe eingesetzt werden.

BESCHREIBUNG DER ABBILDUNGEN

Abb. 1: Schematische Darstellung des VP1/2 kodierenden Bereichs von Parvovirus B19 mit den für die Amplifizierung verwendeten Primer-Sequenzen

Im oberen Teil ist der Aufbau des einzelsträngigen B19-Genoms mit den inversen Regionen an den Enden (Doppelstrang) sowie mit kodierenden Bereichen schematisch dargestellt. Im linken Bereich ist die kodierende Region für die Nicht-Strukturproteine (NS), die als Polypeptid synthetisiert und dann prozessiert werden. Der rechte Bereich kodiert für die Oberflächenproteine des viralen Kapsids (VP1/2), wobei VP1 bis auf einen zusätzlichen N-terminalen Bereich (schraffierter Balken) identisch mit VP2 (schwarzer Balken) ist. Darunter sind die Regionen der Oligodesoxynukleotide O-1 bis O-4 angegeben, die als Primer für die Amplifizierung (PCR) der dazwischen liegenden B19-Sequenzen verwendet wurden (O-1 und O-2 für den VP1-Bereich, bzw O-3 und O-4 für VP-2).

Im unteren Teil der Abbildung sind die DNA-Sequenzen der entsprechenden B19-Bereiche sowie der Oligodesoxynukleotide angegeben. Die Oligodesoxynukleotid-Sequenzenzen sind durch Fettdruck kenntlich gemacht, nicht-homologe Bereiche, d.h. Sequenzen, die nicht mit B19 hybridisieren, sind durch einen Zeilenabstand abgesetzt. Bei O-1 stellen diese nichthybridisierenden Sequenzen Restriktionsenzym-Stellen für EcoRI (GAATTC) und BclI (TGATCA) dar, bei O-3 für EcoRI, BclI und BspHII (TC-ATGA), bei O-4 für HindIII (AAGCT-T). Das amplifizierte VP2 kodierende Fragment (O-3 und O-4) wurde vor dem Einsetzen in pUC-Vektoren mit EcoRI und HindIII verdaut, das VP1 kodierende Fragment mit EcoRI und PstI, wobei die PstI-Schnittstelle in der B19-DNA liegt (ab Position-Nr. 4 der angegebenen Sequenz für O-2, CTGCAG).

Abb. 2 Aminosäure-Sequenzen der in Beispiel 2 beschriebenen Antigene, die rekombinant in E.coli-Zellen produziert werden.

Bedingt durch Klonierungsschritte sind an den N-Termini sowie an den C-Termini (bis auf PANSE UND VP-2) jeweils einige nicht-B19-authentische Fremdaminosäuren mit enthalten, die durch Fettdruck hervor gehoben sind.

Beschrieben werden die Aminosäuresequenzen der in Beispiel 2 beschriebenen Antigene:

Abb. 2-1:      PAN-1
Abb. 2-2:      PCE
Abb. 2-3:      PAN-2
Abb. 2-4:      PAN-3
Abb. 2-5:      PAN-4
Abb. 2-6:      VP2
Abb. 2-7:      PANSE
Abb. 2-8:      PAPST
Abb. 2-9:      PAV-1B
Abb. 2-10:     PAV-1N
Abb. 3:          Schematische Darstellung der Anordnung einiger Peptide zueinander

NS    VP-1/2

0-1  0-5    0-2

0-3                    0-4

**O-1:**

5'GTG AAT TCT GAT CAT

ATG AGT AAA AAA AGT GGC AAA TGG3'

5'AAA GCT TTG TAG ATT ATG AGT AAA AAA AGT GGC AAA TGG TGG3'

3'TTT CGA AAC ATC TAA TAC TCA TTT TTT TCA CCG TTT ACC ACC5'

**O-2:**

5'ATT CTG CAG AAG CCA GCA CTG GTG CAG GAG GGG GGG GCA3'

3'TAA GAC GTC TTC GGT CGT GAC CAC GTC CTC CCC CCC CGT5'

3'C TTC GGT CGT GAC CAC GTC CTC CCC5'

**O-3:**

5'G AGG AAT TCT CTG ATC

ATG ACT TCA GTT AAT TCT GCA GAA GCC3'

5'A GAA AAA TAC CCA AGC ATG ACT TCA GTT AAT TCT GCA GAA GCC3'

3'T CTT TTT ATG GGT TCG TAC TGA AGT CAA TTA AGA CGT CTT CGG5'

**O-4:**

5'TTG TAA ACA CTC CCC ACC GTG CCC TCA GCC AGG ATG CGT A3'

3'AAC ATT TGT GAG GGG TCC CAC GGG AGT CGG TCC TAC GCA T5'

3'GAG GGG TGG CAC GGG AGT CGG TCC T

TC GAA GAG5'

**O-5**

5'G CTA CAA GCT GGG CCC CCG CAA AG3'    -------

5'GAG CTA CAA GCT GGG CCC CCG CAA AGT GCT GTT GAC AGT GCT3'

3'CAC GAT GTT CGA CCC GGG GGC GTT TCA CGA CAA CTG TCA CGA5'

AAB. 1

Abb. 2

**Abb. 2-1**

**PAN-1:**

Met Ser Lys Lys Ser Gly Lys Trp Trp Glu Ser Asp Asp Lys Phe Ala Lys Ala Val Tyr Gln Gln Phe Val Glu Phe Tyr Glu Lys Val Thr Gly Thr Asp Leu Glu Leu Ile Gln Ile Leu Lys Asp His Tyr Asn Ile Ser Leu Asp Asn Pro Leu Glu Asn Pro Ser Ser Leu Phe Asp Leu Val Ala Arg Ile Lys Asn Asn Leu Lys Asn Ser Pro Asp Leu Tyr Ser His His Phe Gln Ser His Gly Gln Leu Ser Asp His Pro His Ala Leu Ser Ser Ser Ser Ser His Ala Glu Pro Arg Gly Glu Asn Ala Val Leu Ser Ser Glu Asp Leu His Lys Pro Gly Gln Val Ser Val Gln Leu Pro Gly Thr Asn Tyr Val Gly Pro Gly Asn Glu Leu Gln Ala Gly Pro Pro Gln Ser Ala Val Asp Ser Ala Ala Arg Ile His Asp Phe Arg Tyr Ser Gln Leu Ala Lys Leu Gly Ile Asn Pro Tyr Thr His Trp Thr Val Ala Asp Glu Glu Leu Leu Lys Asn Ile Lys Asn Glu Thr Gly Phe Gln Ala Gln Val Val Lys Asp Tyr Phe Thr Leu Lys Gly Ala Ala Ala Pro Val Ala His Phe Gln Gly Ser Leu Pro Glu Val Pro Ala Tyr Asn Ala Ser Glu Lys Tyr Pro Ser

**Abb. 2-2**

**PCE:**

Gluthation-S-Transferase - His Met Ser Lys Lys Ser Gly Lys Trp Trp Glu Ser Asp Asp Lys Phe Ala Lys Ala Val Tyr Gln Gln Phe Val Glu Phe Tyr Glu Lys Val Thr Gly Thr Asp Leu Glu Leu Ile Gln Ile Leu Lys Asp His Tyr Asn Ile Ser Leu Asp Asn Pro Leu Glu Asn Pro Ser Ser Leu Phe Asp Leu Val Ala Arg Ile Lys Asn Asn Leu Lys Asn Ser Pro Asp Leu Tyr Ser His His Phe Gln Ser His Gly Gln Leu Ser Asp His Pro His Ala Leu Ser Ser Ser Ser Ser His Ala Glu Pro Arg Gly Glu Asn Ala Val Leu Ser Ser Glu Asp Leu His Lys Pro Gly Gln Val Ser Val Gln Leu Pro Gly Thr Asn Tyr Val Gly Pro Gly Asn Glu Leu Gln Ala Gly Pro Pro Gln Ser Ala Val Asp Ser Ala Ala Arg Ile His Asp Phe Arg Tyr Ser Gln Leu Ala Lys Leu Gly Ile Asn Pro Tyr Thr His Trp Thr Val Ala Asp Glu Glu Leu Leu Lys Asn Ile Lys Asn Glu Thr Gly Phe Gln Ala Gln Val Val Lys Asp Tyr Phe Thr Leu Lys Gly Ala Gly Glu Phe Ile Val Thr Asp

**Abb. 2-3**

**PAN-2**

Gly Ser Arg Arg Pro Asp His Met Ser Lys Lys Ser Gly Lys Trp Trp Glu Ser Asp Asp Lys Phe Ala Lys Ala Val Tyr Gln Gln Phe Val Glu Phe Tyr Glu Lys Val Thr Gly Thr Asp Leu Glu Leu Ile Gln Ile Leu Lys Asp His Tyr Asn Ile Ser Leu Asp Asn Pro Leu Glu Asn Pro Ser Ser Leu Phe Asp Leu Val Ala Arg Ile Lys Asn Asn Leu Lys Asn Ser Pro Asp Leu Tyr Ser His His Phe Gln Ser His Gly Gln Leu Ser Asp His Pro His Ala Leu Ser Ser Ser Ser Ser His Ala Glu Pro Arg Gly Glu Asn Ala Val Leu Ser Ser Glu Asp Leu His Lys Pro Gly Gln Val Ser Val Gln Leu Pro Gly Thr Asn Tyr Val Gly Pro Gly Asn Glu Leu Gln Ala Gly Pro Pro Gln Ser Ala Val Gly Asp Pro Arg Glu Phe Ile Val Thr Asp

EP 0 514 413 B1

Abb. 2-4

PAN-3

Gly Ile Leu Ser Arg Arg Pro Asp His Met Ser Lys Lys Ser Gly Lys Trp Trp Glu Ser Asp Asp Lys Phe Ala Lys Ala Val Tyr Gln Gln Phe Val Glu Phe Tyr Glu Lys Val Thr Gly Thr Asp Leu Glu Leu Ile Gln Ile Leu Lys Asp His Tyr Asn Ile Ser Leu Asp Asn Pro Leu Glu Asn Pro Ser Ser Leu Phe Asp Leu Val Ala Arg Ile Lys Asn Asn Leu Lys Asn Ser Pro Asp Leu Tyr Ser His His Phe Gln Ser His Gly Gln Leu Ser Asp His Pro His Ala Leu Ser Ser Ser Ser His Ala Glu Pro Arg Gly Glu Asn Ala Val Leu Ser Ser Glu Asp Leu His Lys Pro Gly Gln Val Ser Val Gln Leu Pro Gly Thr Asn Tyr Val Gly Pro Gly Asn Glu Leu Gln Ala Gly Pro Pro Gln Ser Ala Val Gly Asp Pro Leu Glu Asp Pro Arg Val Pro Ser Ser Asn Ser

Abb. 2-5

PAN-4

Gly Ser Arg Arg Pro Asp His Met Ser Lys Lys Ser Gly Lys Trp Trp Glu Ser Asp Asp Lys Phe Ala Lys Ala Val Tyr Gln Gln Phe Val Glu Phe Tyr Glu Lys Val Thr Gly Thr Asp Leu Glu Leu Ile Gln Ile Leu Lys Asp His Tyr Asn Ile Ser Leu Asp Asn Pro Leu Glu Asn Pro Ser Ser Leu Phe Asp Leu Val Ala Arg Ile Lys Asn Asn Leu Lys Asn Ser Pro Asp Leu Tyr Ser His His Phe Gln Ser His Gly Gln Leu Ser Asp His Pro His Ala Leu Ser Ser Ser Ser Ser His Ala Glu Pro Arg Gly Glu Asn Ala Val Leu Ser Ser Glu Asp Leu His Lys Pro Gly Gln Val Ser Val Gln Leu Pro Gly Thr Asn Tyr Val Gly Pro Gly Asn Glu Leu Gln Ala Gly Pro Pro Gln Ser Ala Val Asp Ser Ala Ala Arg Ile His Asp Phe Arg Tyr Ser Gln Leu Ala Lys Leu Gly Ile Asn Pro Tyr Thr His Trp Thr Val Ala Asp Glu Glu Leu Leu Lys Asn Ile Lys Asn Glu Thr Gly Phe Gln Ala Gln Val Val Lys Asp Tyr Phe Thr Leu Lys Gly Ala Ala Ala Pro Val Ala His Phe Gln Gly Ser Leu Pro Glu Val Pro Ala Tyr Asn Ala Ser Glu Lys Tyr Pro Ser Met Thr Ser Val Asn Ser Ala Gly Arg Arg Ile Pro Gly Asn Ser Ser

Abb. 2-6

VP2:

Met Thr Met Ile Thr Asn Ser Leu Ile Met Thr Ser Val Asn Ser Ala Glu Ala Ser Thr Gly Ala Gly Gly Gly Gly Ser Asn Ser Val Lys Ser Met Trp Ser Glu Gly Ala Thr Phe Ser Ala Asn Ser Val Thr Cys Thr Phe Ser Arg Gln Phe Leu Ile Pro Tyr Asp Pro Glu His His Tyr Lys Val Phe Ser Pro Ala Ala Ser Ser Cys His Asn Ala Ser Gly Lys Glu Ala Lys Val Cys Thr Ile Ser Pro Ile Met Gly Tyr Ser Thr Pro Trp Arg Tyr Leu Asp Phe Asn Ala Leu Asn Leu Phe Phe Ser Pro Leu Glu Phe Gln His Leu Ile Glu Asn Tyr Gly Ser Ile Ala Pro Asp Ala Leu Thr Val Thr Ile Ser Glu Ile Ala Val Lys Asp Val Thr Asp Lys Thr Gly Gly Gly Val Gln Val Thr Asp Ser Thr Thr Gly Arg Leu Cys Met Leu Val Asp His Glu Tyr Lys Tyr Pro Tyr Val Leu Gly Gln Gly Gln Asp Thr Leu Ala Pro Glu Leu Pro Ile Trp Val Tyr Phe Pro Pro Gln Tyr Ala Tyr Leu Thr Val Gly Asp Val Asn Thr Gln Gly Ile Ser Gly Asp Ser Lys Lys Leu Ala Ser Glu Glu Ser Ala Phe Tyr Val Leu Glu His Ser Ser Phe Gln Leu Leu Gly Thr Gly Gly Thr Ala Ser Met Ser Tyr Lys Phe Pro Pro Val Pro Pro Glu Asn Leu Glu Gly Cys Ser Gln His Phe Tyr Glu Met Tyr Asn Pro Leu Tyr Gly Ser Arg Leu Gly Val Pro Asp Thr Leu Gly Gly Asp Pro Lys Phe Arg Ser Leu Thr His Glu Asp His Ala Ile Gln Pro Gln Asn Phe Met Pro Gly Pro Leu Val Asn Ser Val Ser Thr Lys Glu Gly Asp Ser Ser Asn Thr Gly Ala Gly Lys Ala Leu Thr Gly Leu Ser Thr Gly Thr Ser Gln Asn Thr Arg Ile Ser Leu Arg Pro Gly Pro Val Ser Gln Pro Tyr His His Trp Asp Thr Asp Lys Tyr Val Thr Gly Ile Asn Ala Ile Ser His Gly Gln Thr Thr Tyr Gly Asn Ala Glu Asp Lys Glu Tyr Gln Gln Gly Val Gly Arg Phe Pro Asn Glu Lys Glu Gln Leu Lys Gln Leu Gln Gly Leu Asn Met His Thr Tyr Phe Pro Asn Lys Gly Thr Gln Gln Tyr Thr Asp Gln Ile Glu Arg Pro Leu Met Val Gly Ser Val Trp Asn Arg Arg Ala Leu His Tyr Glu Ser Gln Leu Trp Ser Lys Ile Pro Asn Leu Asp Asp Ser Phe Lys Thr Gln Phe Ala Ala Leu Gly Gly Trp Gly Leu His Gln Pro Pro Pro Gln Ile Phe Leu Lys Gln Tyr Ala Val Gly Ile Met Thr Val Thr Met Thr Phe Lys Leu Gly Pro Arg Lys Ala Thr Gly Arg Trp Asn Pro Gln Pro Gly Val Tyr Pro Pro His Ala Ala Gly His Leu Pro Tyr Val Leu Tyr Asp Pro Thr Ala Thr Asp Ala Lys Gln His His Arg His Gly Tyr Glu Lys Pro Glu Glu Leu Trp Thr Ala Lys Ser Arg Val His Pro Leu

15

Abb. 2-7

PANSE:

Met Thr Met Ile Thr Pro Ser Leu His Ala Cys Met Leu Val Asp His Glu Tyr Lys Tyr Pro Tyr Val Leu Gly
Gln Gly Gln Asp Thr Leu Ala Pro Glu Leu Pro Ile Trp Val Tyr Phe Pro Pro Gln Tyr Ala Tyr Leu Thr Val
Gly Asp Val Asn Thr Gln Gly Ile Ser Gly Asp Ser Lys Lys Leu Ala Ser Glu Glu Ser Ala Phe Tyr Val Leu
Glu His Ser Ser Phe Gln Leu Leu Gly Thr Gly Gly Thr Ala Ser Met Ser Tyr Lys Phe Pro Pro Val Pro Pro
Glu Asn Leu Glu Gly Cys Ser Gln His Phe Tyr Glu Met Tyr Asn Pro Leu Tyr Gly Ser Arg Leu Gly Val Pro
Asp Thr Leu Gly Gly Asp Pro Lys Phe Arg Ser Leu Thr His Glu Asp His Ala Ile Gln Pro Gln Asn Phe Met
Pro Gly Pro Leu Val Asn Ser Val Ser Thr Lys Glu Gly Asp Ser Ser Asn Thr Gly Ala Gly Lys Ala Leu Thr
Gly Leu Ser Thr Gly Thr Ser Gln Asn Thr Arg Ile Ser Leu Arg Pro Gly Pro Val Ser Gln Pro Tyr His His
Trp Asp Thr Asp Lys Tyr Val Thr Gly Ile Asn Ala Ile Ser His Gly Gln Thr Thr Tyr Gly Asn Ala Glu Asp
Lys Glu Tyr Gln Gln Gly Val Gly Arg Phe Pro Asn Glu Lys Glu Gln Leu Lys Gln Leu Gln Gly Leu Asn
Met His Thr Tyr Phe Pro Asn Lys Gly Thr Gln Gln Tyr Thr Asp Gln Ile Glu Arg Pro Leu Met Val Gly Ser
Val Trp Asn Arg Arg Ala Leu His Tyr Glu Ser Gln Leu Trp Ser Lys Ile Pro Asn Leu Asp Asp Ser Phe Lys
Thr Gln Phe Ala Ala Leu Gly Gly Trp Gly Leu His Gln Pro Pro Pro Gln Ile Phe Leu Lys Gln Tyr Ala Val
Gly Ile Met Thr Val Thr Met Thr Phe Lys Leu Gly Pro Arg Lys Ala Thr Gly Arg Trp Asn Pro Gln Pro Gly
Val Tyr Pro Pro His Ala Ala Gly His Leu Pro Tyr Val Leu Tyr Asp Pro Thr Ala Thr Asp Ala Lys Gln His
His Arg His Gly Tyr Glu Lys Pro Glu Glu Leu Trp Thr Ala Lys Ser Arg Val His Pro Leu

Abb. 2-8

PAPST:

Met Thr Met Ile Thr Pro Ser Leu Ala Ala Glu Ala Ser Thr Gly Ala Gly Gly Gly Gly Ser Asn Ser Val Lys
Ser Met Trp Ser Glu Gly Ala Thr Phe Ser Ala Asn Ser Val Thr Cys Thr Phe Ser Arg Gln Phe Leu Ile Pro
Tyr Asp Pro Glu His His Tyr Lys Val Phe Ser Pro Ala Ala Ser Ser Cys His Asn Ala Ser Gly Lys Glu Ala
Lys Val Cys Thr Ile Ser Pro Ile Met Gly Tyr Ser Thr Pro Trp Arg Tyr Leu Asp Phe Asn Ala Leu Asn Leu
Phe Phe Ser Pro Leu Glu Phe Gln His Leu Ile Glu Asn Tyr Gly Ser Ile Ala Pro Asp Ala Leu Thr Val Thr
Ile Ser Glu Ile Ala Val Lys Asp Val Thr Asp Lys Thr Gly Gly Gly Val Gln Val Thr Asp Ser Thr Thr Gly
Arg Leu Cys Met Leu Val Asp His Glu Tyr Lys Tyr Pro Tyr Val Leu Gly Gln Gly Gln Asp Thr Leu Ala Pro
Glu Leu Pro Ile Trp Val Tyr Phe Pro Pro Gln Tyr Ala Tyr Leu Thr Val Gly Asp Val Asn Thr Gln Gly Ile
Ser Gly Asp Ser Lys Lys Leu Ala Ser Glu Glu Ser Ala Phe Tyr Val Leu Glu His Ser Ser Phe Gln Leu Leu
Gly Thr Gly Gly Thr Ala Ser Met Ser Tyr Lys Phe Pro Pro Val Pro Pro Glu Asn Leu Glu Gly Cys Arg Ser
Thr Asp Pro Arg Glu Phe Thr Gly Arg Arg Phe Thr Thr Ser

Abb. 2-9

PAV-1-B

Met Thr Ile Thr Asn Ser Asp His Met Ser Lys Lys Ser Gly Lys Trp Trp Glu Ser Asp Asp Lys Phe Ala Lys
Ala Val Tyr Gln Gln Phe Val Glu Phe Tyr Glu Lys Val Thr Gly Thr Asp Leu Glu Leu Ile Gln Ile Leu Lys
Asp His Tyr Asn Ile Ser Leu Asp Asn Pro Leu Glu Asn Pro Ser Ser Leu Phe Asp Leu Val Ala Arg Ile Lys
Asn Asn Leu Lys Asn Ser Pro Asp Leu Tyr Ser His His Phe Gln Ser His Gly Gln Leu Ser Asp His Pro His
Ala Leu Ser Ser Ser Ser Ser His Ala Glu Pro Arg Gly Glu Asn Ala Val Leu Ser Ser Glu Asp Leu His Lys
Pro Gly Gln Val Ser Val Gln Leu Pro Gly Thr Asn Tyr Val Gly Pro Gly Asn Glu Leu Gln Ala Gly Pro Pro
Gln Ser Ala Val Asp Ser Ala Ala Arg Ile His Asp Phe Arg Tyr Ser Gln Leu Ala Lys Leu Gly Ile Asn Pro
Tyr Thr His Trp Thr Val Ala Asp Glu Glu Leu Leu Lys Asn Ile Lys Asn Glu Thr Gly Phe Gln Ala Gln Val
Val Lys Asp Tyr Phe Thr Leu Lys Gly Ala Ala Ala Pro Val Ala His Phe Gln Gly Ser Leu Pro Glu Val Pro
Ala Tyr Asn Ala Ser Glu Lys Tyr Pro Ser Met Thr Ser Val Asn Ser Ala Glu Ala Ser Thr Gly Ala Gly Gly
Gly Gly Ser Asn Ser Val Lys Ser Met Trp Ser Glu Gly Ala Thr Phe Ser Ala Asn Ser Val Thr Cys Thr Phe
Ser Arg Gln Phe Leu Ile Pro Tyr Asp Pro Glu His His Tyr Lys Val Phe Ser Pro Ala Ala Ser Ser Cys His
Asn Ala Ser Gly Lys Glu Ala Lys Val Cys Thr Ile Ser Pro Ile Met Gly Tyr Ser Thr Pro Trp Arg Tyr Leu Asp
Phe Asn Ala Leu Asn Leu Phe Phe Ser Pro Leu Glu Phe Gln His Leu Ile Glu Asn Tyr Gly Ser Ile Ala Pro
Asp Ala Leu Thr Val Thr Ile Ser Glu Ile Ala Val Lys Asp Val Thr Asp Lys Thr Gly Gly Gly Val Gln Val
Thr Asp Ser Thr Thr Gly Arg Leu Cys Met Leu Val Asp His Glu Tyr Lys Tyr Pro Tyr Val Leu Gly Gln Gly
Gln Asp Thr Leu Ala Pro Glu Leu Pro Ile Trp Val Tyr Phe Pro Pro Gln Tyr Ala Tyr Leu Thr Val Gly Asp
Val Asn Thr Gln Gly Ile Ser Gly Asp Ser Lys Lys Leu Ala Ser Glu Glu Ser Ala Phe Tyr Val Leu Glu His
Ser Ser Phe Gln Leu Leu Gly Thr Gly Gly Thr Ala Ser Met Ser Tyr Lys Phe Pro Pro Val Pro Pro Glu Asn
Leu Glu Gly Cys Ser Gln His Phe Tyr Glu Met Tyr Asn Pro Leu Tyr Gly Ser Ser Arg Val Asp Leu Gln

Abb.2-10

PAV-1-N

Met Thr Ile Thr Asn Ser Asp His Met Ser Lys Lys Ser Gly Lys Trp Trp Glu Ser Asp Asp Lys Phe Ala Lys
Ala Val Tyr Gln Gln Phe Val Glu Phe Tyr Glu Lys Val Thr Gly Thr Asp Leu Glu Leu Ile Gln Ile Leu Lys
Asp His Tyr Asn Ile Ser Leu Asp Asn Pro Leu Glu Asn Pro Ser Ser Leu Phe Asp Leu Val Ala Arg Ile Lys
Asn Asn Leu Lys Asn Ser Pro Asp Leu Tyr Ser His His Phe Gln Ser His Gly Gln Leu Ser Asp His Pro His
Ala Leu Ser Ser Ser Ser Ser His Ala Glu Pro Arg Gly Glu Asn Ala Val Leu Ser Ser Glu Asp Leu His Lys
Pro Gly Gln Val Ser Val Gln Leu Pro Gly Thr Asn Tyr Val Gly Pro Gly Asn Glu Leu Gln Ala Gly Pro Pro
Gln Ser Ala Val Asp Ser Ala Ala Arg Ile His Asp Phe Arg Tyr Ser Gln Leu Ala Lys Leu Gly Ile Asn Pro
Tyr Thr His Trp Thr Val Ala Asp Glu Glu Leu Leu Lys Asn Ile Lys Asn Glu Thr Gly Phe Gln Ala Gln Val
Val Lys Asp Tyr Phe Thr Leu Lys Gly Ala Ala Ala Pro Val Ala His Phe Gln Gly Ser Leu Pro Glu Val Pro
Ala Tyr Asn Ala Ser Glu Lys Tyr Pro Ser Met Thr Ser Val Asn Ser Ala Glu Ala Ser Thr Gly Ala Gly Gly
Gly Gly Ser Asn Ser Val Lys Ser Met Trp Ser Glu Gly Ala Thr Phe Ser Ala Asn Ser Val Thr Cys Thr Phe
Ser Arg Gln Phe Leu Ile Pro Tyr Asp Pro Glu His His Tyr Lys Val Phe Ser Pro Ala Ala Ser Ser Cys His
Asn Ala Ser Gly Lys Glu Ala Lys Val Cys Thr Ile Ser Pro Ile Met Gly Tyr Ser Thr Pro Trp Arg Tyr Leu Asp
Phe Asn Ala Leu Asn Leu Phe Phe Ser Pro Leu Glu Phe Gln His Leu Ile Glu Asn Tyr Gly Ser Ile Ala Pro
Asp Ala Leu Thr Val Thr Ile Ser Glu Ile Ala Val Lys Asp Val Thr Asp Lys Thr Gly Gly Gly Val Gln Val
Thr Asp Ser Thr Thr Gly Arg Leu Cys Ser Asn

VP-1    VP-2

PAN 1 ▬▬▬▬▬▬

PCE 1 ▬▬▬▬▬

PAN 2 ▬▬▬▬

PAN 3 ▬▬▬▬

PAN 4 ▬▬▬▬▬▬

**VP-2** ▬▬▬▬▬▬▬▬▬▬

**PANSE** ▬▬▬▬▬▬▬▬

**PAPST** ▬▬▬▬▬

Ausschnitt  (VP1-VP2)

227

PAPEP1   PAPEP2  PAPEP3  PAPEP4   PAPEP5   PAPEP6        PAPEP7

PAPEP 8

**Abb. 3**

**Patentansprüche**

1. Immunologisch aktives Peptid oder Polypeptid, das einen Teil der Aminosäuresequenz der Kapsidproteine VP1 oder VP2 des Parvovirus B19 aufweist, dadurch gekennzeichnet, daß es frei ist von Verunreinigungen, die den Nachweis von Parvovirus B19 spezifischen Antikörpern stören können und das Polypeptid, das eine Teilsequenz von 8 bis 50 Aminosäureresten, insbesondere 10 bis 32 Aminosäureresten des Peptids PAN1, wie in Abb. 2-1 dargestellt, ist, oder die Aminosäuresequenz

Asn Pro Tyr Thr His Trp Thr Val Ala Asp Glu Glu Leu Leu Lys His
Ile Lys und/oder

Ser Lys Lys Ser Gly Lys Trp Trp Glu Ser Asp Asp Lys Phe Ala Lys
Ala Val Tyr und/oder

Leu Lys Asp His Tyr Asn Ile Ser Leu Asp Asn Pro Leu Glu Asn Pro
Ser Ser und/oder

Ile Lys Asn Asn Leu Lys Asn Ser Pro Asp Leu Tyr Ser His His Phe
Gln Ser His Gly Gln Leu Ser Asp His Pro His Ala und/oder

Ser Ser His Ala Glu Pro Arg Gly Glu Asn Ala Val Leu Ser Ser Glu
Asp Leu His Lys Pro Gly Gln Val und/oder

Asn Tyr Val Gly Pro Gly Asn Glu Leu Gln Ala Gly Pro Pro Gln Ser
Ala Val Asp Ser Ala Ala Arg Ile His Asp Phe Arg Tyr Ser Gln Leu

und/oder

Pro Tyr Thr His Trp Thr Val Ala Asp Glu Glu Leu Leu Lys Asn Ile
Lys Asn Glu Thr Gly Phe und/oder

Asn Ala Ser Glu Lys Tyr Pro Ser Met Thr Ser Val Asn Ser Ala Glu
Ala Ser

aufweist und/oder die Aminosäuresequenz

```
His Met Ser Lys Lys Ser Gly Lys Trp Trp Glu Ser Asp Asp Lys Phe
Ala Lys Ala Val Tyr Gln Gln Phe Val Glu Phe Tyr Glu Lys Val Thr
Gly Thr Asp Leu Glu Leu Ile Gln Ile Leu Lys Asp His Tyr Asn Ile
Ser Leu Asp Asn Pro Leu Glu Asn Pro Ser Ser Leu Phe Asp Leu Val
Ala Arg Ile Lys Asn Asn Leu Lys Asn Ser Pro Asp Leu Tyr Ser His
His Phe Gln Ser His Gly Gln Leu Ser Asp His Pro His Ala Leu Ser
Ser Ser Ser Ser His Ala Glu Pro Arg Gly Glu Asn Ala Val Leu Ser
Ser Glu Asp Leu His Lys Pro Gly Gln Val Ser Val Gln Leu Pro Gly
Thr Asn Tyr Val Gly Pro Gly Asn Glu Leu Gln Ala Gly Pro Pro Gln
Ser Ala Val Asp Ser Ala Ala Arg Ile His Asp Phe Arg Tyr Ser Gln
Leu Ala Lys Leu Gly Ile Asn Pro Tyr Thr His Trp Thr Val Ala Asp
Glu Glu Leu Leu Lys Asn Ile Lys Asn Glu Thr Gly Phe Gln Ala Gln
Val Val Lys Asp Tyr Phe Thr Leu Lys Gly Ala Gly Glu Phe Ile Val
Thr Asp und/oder


Gly Ser Arg Arg Pro Asp His Met Ser Lys Lys Ser Gly Lys Trp Trp
Glu Ser Asp Asp Lys Phe Ala Lys Ala Val Tyr Gln Gln Phe Val Glu
Phe Tyr Glu Lys Val Thr Gly Thr Asp Leu Glu Leu Ile Gln Ile Leu
Lys Asp His Tyr Asn Ile Ser Leu Asp Asn Pro Leu Glu Asn Pro Ser
Ser Leu Phe Asp Leu Val Ala Arg Ile Lys Asn Asn Leu Lys Asn Ser
Pro Asp Leu Tyr Ser His His Phe Gln Ser His Gly Gln Leu Ser Asp
His Pro His Ala Leu Ser Ser Ser Ser Ser His Ala Glu Pro Arg Gly
Glu Asn Ala Val Leu Ser Ser Glu Asp Leu His Lys Pro Gly Gln Val
Ser Val Gln Leu Pro Gly Thr Asn Tyr Val Gly Pro Gly Asn Glu Leu

Gln Ala Gly Pro Pro Gln Ser Ala Val Gly Asp Pro Arg Glu Phe Ile
Val Thr Asp und/oder


Gly Ile Leu Ser Arg Arg Pro Asp His Met Ser Lys Lys Ser Gly Lys
Trp Trp Glu Ser Asp Asp Lys Phe Ala Lys Ala Val Tyr Gln Gln Phe
Val Glu Phe Tyr Glu Lys Val Thr Gly Thr Asp Leu Glu Leu Ile Gln
Ile Leu Lys Asp His Tyr Asn Ile Ser Leu Asp Asn Pro Leu Glu Asn
Pro Ser Ser Leu Phe Asp Leu Val Ala Arg Ile Lys Asn Asn Leu Lys
Asn Ser Pro Asp Leu Tyr Ser His His Phe Gln Ser His Gly Gln Leu
Ser Asp His Pro His Ala Leu Ser Ser Ser Ser Ser His Ala Glu Pro
Arg Gly Glu Asn Ala Val Leu Ser Ser Glu Asp Leu His Lys Pro Gly
Gln Val Ser Val Gln Leu Pro Gly Thr Asn Tyr Val Gly Pro Gly Asn
Glu Leu Gln Ala Gly Pro Pro Gln Ser Ala Val Gly Asp Pro Leu Glu
Asp Pro Arg Val Pro Ser Ser Asn Ser und/oder
```

```
Gly Ser Arg Arg Pro Asp His Met Ser Lys Lys Ser Gly Lys Trp Trp
Glu Ser Asp Asp Lys Phe Ala Lys Ala Val Tyr Gln Gln Phe Val Glu
Phe Tyr Glu Lys Val Thr Gly Thr Asp Leu Glu Leu Ile Gln Ile Leu
Lys Asp His Tyr Asn Ile Ser Leu Asp Asn Pro Leu Glu Asn Pro Ser
Ser Leu Phe Asp Leu Val Ala Arg Ile Lys Asn Asn Leu Lys Asn Ser
Pro Asp Leu Tyr Ser His His Phe Gln Ser His Gly Gln Leu Ser Asp
His Pro His Ala Leu Ser Ser Ser Ser Ser His Ala Glu Pro Arg Gly
Glu Asn Ala Val Leu Ser Ser Glu Asp Leu His Lys Pro Gly Gln Val
Ser Val Gln Leu Pro Gly Thr Asn Tyr Val Gly Pro Gly Asn Glu Leu
Gln Ala Gly Pro Pro Gln Ser Ala Val Asp Ser Ala Ala Arg Ile His
Asp Phe Arg Tyr Ser Gln Leu Ala Lys Leu Gly Ile Asn Pro Tyr Thr
His Trp Thr Val Ala Asp Glu Glu Leu Leu Lys Asn Ile Lys Asn Glu
Thr Gly Phe Gln Ala Gln Val Val Lys Asp Tyr Phe Thr Leu Lys Gly
Ala Ala Ala Pro Val Ala His Phe Gln Gly Ser Leu Pro Glu Val Pro
Ala Tyr Asn Ala Ser Glu Lys Tyr Pro Ser Met Thr Ser Val Asn Ser
Ala Gly Arg Arg Ile Pro Gly Asn Ser Ser und/oder
```

Met Thr Met Ile Thr Pro Ser Leu His Ala Cys Met Leu Val Asp His
Glu Tyr Lys Tyr Pro Tyr Val Leu Gly Gln Gly Gln Asp Thr Leu Ala
Pro Glu Leu Pro Ile Trp Val Tyr Phe Pro Pro Gln Tyr Ala Tyr Leu
Thr Val Gly Asp Val Asn Thr Gln Gly Ile Ser Gly Asp Ser Lys Lys
Leu Ala Ser Glu Glu Ser Ala Phe Tyr Val Leu Glu His Ser Ser Phe
Gln Leu Leu Gly Thr Gly Gly Thr Ala Ser Met Ser Tyr Lys Phe Pro
Pro Val Pro Pro Glu Asn Leu Glu Gly Cys Ser Gln His Phe Tyr Glu
Met Tyr Asn Pro Leu Tyr Gly Ser Arg Leu Gly Val Pro Asp Thr Leu
Gly Gly Asp Pro Lys Phe Arg Ser Leu Thr His Glu Asp His Ala Ile
Gln Pro Gln Asn Phe Met Pro Gly Pro Leu Val Asn Ser Val Ser Thr
Lys Glu Gly Asp Ser Ser Asn Thr Gly Ala Gly Lys Ala Leu Thr Gly
Leu Ser Thr Gly Thr Ser Gln Asn Thr Arg Ile Ser Leu Arg Pro Gly
Pro Val Ser Gln Pro Tyr His His Trp Asp Thr Asp Lys Tyr Val Thr
Gly Ile Asn Ala Ile Ser His Gly Gln Thr Thr Tyr Gly Asn Ala Glu
Asp Lys Glu Tyr Gln Gln Gly Val Gly Arg Phe Pro Asn Glu Lys Glu
Gln Leu Lys Gln Leu Gln Gly Leu Asn Met His Thr Tyr Phe Pro Asn
Lys Gly Thr Gln Gln Tyr Thr Asp Gln Ile Glu Arg Pro Leu Met Val
Gly Ser Val Trp Asn Arg Arg Ala Leu His Tyr Glu Ser Gln Leu Trp
Ser Lys Ile Pro Asn Leu Asp Asp Ser Phe Lys Thr Gln Phe Ala Ala
Leu Gly Gly Trp Gly Leu His Gln Pro Pro Pro Gln Ile Phe Leu Lys
Gln Tyr Ala Val Gly Ile Met Thr Val Thr Met Thr Phe Lys Leu Gly
Pro Arg Lys Ala Thr Gly Arg Trp Asn Pro Gln Pro Gly Val Tyr Pro
Pro His Ala Ala Gly His Leu Pro Tyr Val Leu Tyr Asp Pro Thr Ala
Thr Asp Ala Lys Gln His His Arg His Gly Tyr Glu Lys Pro Glu Glu
Leu Trp Thr Ala Lys Ser Arg Val His Pro Leu und/oder

```
Met Thr Met Ile Thr Pro Ser Leu Ala Ala Glu Ala Ser Thr Gly Ala
Gly Gly Gly Gly Ser Asn Ser Val Lys Ser Met Trp Ser Glu Gly Ala
Thr Phe Ser Ala Asn Ser Val Thr Cys Thr Phe Ser Arg Gln Phe Leu
Ile Pro Tyr Asp Pro Glu His His Tyr Lys Val Phe Ser Pro Ala Ala
Ser Ser Cys His Asn Ala Ser Gly Lys Glu Ala Lys Val Cys Thr Ile
Ser Pro Ile Met Gly Tyr Ser Thr Pro Trp Arg Tyr Leu Asp Phe Asn
Ala Leu Asn Leu Phe Phe Ser Pro Leu Glu Phe Gln His Leu Ile Glu
Asn Tyr Gly Ser Ile Ala Pro Asp Ala Leu Thr Val Thr Ile Ser Glu
Ile Ala Val Lys Asp Val Thr Asp Lys Thr Gly Gly Gly Val Gln Val
Thr Asp Ser Thr Thr Gly Arg Leu Cys Met Leu Val Asp His Glu Tyr
Lys Tyr Pro Tyr Val Leu Gly Gln Gly Gln Asp Thr Leu Ala Pro Glu
Leu Pro Ile Trp Val Tyr Phe Pro Pro Gln Tyr Ala Tyr Leu Thr Val
Gly Asp Val Asn Thr Gln Gly Ile Ser Gly Asp Ser Lys Lys Leu Ala
Ser Glu Glu Ser Ala Phe Tyr Val Leu Glu His Ser Ser Phe Gln Leu
Leu Gly Thr Gly Gly Thr Ala Ser Met Ser Tyr Lys Phe Pro Pro Val
Pro Pro Glu Asn Leu Glu Gly Cys Arg Ser Thr Asp Pro Arg Glu Phe
Thr Gly Arg Arg Phe Thr Thr Ser und/oder

Met Ser Lys Lys Ser Gly Lys Trp Trp Glu Ser Asp Asp Lys Phe Ala
Lys Ala Val Tyr Gln Gln Phe Val Glu Phe Tyr Glu Lys Val Thr Gly
Thr Asp Leu Glu Leu Ile Gln Ile Leu Lys Asp His Tyr Asn Ile Ser
Leu Asp Asn Pro Leu Glu Asn Pro Ser Ser Leu Phe Asp Leu Val Ala
Arg Ile Lys Asn Asn Leu Lys Asn Ser Pro Asp Leu Tyr Ser His His
Phe Gln Ser His Gly Gln Leu Ser Asp His Pro His Ala Leu Ser Ser
Ser Ser Ser His Ala Glu Pro Arg Gly Glu Asn Ala Val Leu Ser Ser
Glu Asp Leu His Lys Pro Gly Gln Val Ser Val Gln Leu Pro Gly Thr
Asn Tyr Val Gly Pro Gly Asn Glu Leu Gln Ala Gly Pro Pro Gln Ser
Ala Val Asp Ser Ala Ala Arg Ile His Asp Phe Arg Tyr Ser Gln Leu
Ala Lys Leu Gly Ile Asn Pro Tyr Thr His Trp Thr Val Ala Asp Glu
Glu Leu Leu Lys Asn Ile Lys Asn Glu Thr Gly Phe Gln Ala Gln Val
Val Lys Asp Tyr Phe Thr Leu Lys Gly Ala Ala Ala Pro Val Ala His
Phe Gln Gly Ser Leu Pro Glu Val Pro Ala Tyr Asn Ala Ser Glu Lys
Tyr Pro Ser und/oder
```

23

Met Thr Ile Thr Asn Ser Asp His Met Ser Lys Lys Ser Gly Lys Trp
Trp Glu Ser Asp Asp Lys Phe Ala Lys Ala Val Tyr Gln Gln Phe Val
Glu Phe Tyr Glu Lys Val Thr Gly Thr Asp Leu Glu Leu Ile Gln Ile
Leu Lys Asp His Tyr Asn Ile Ser Leu Asp Asn Pro Leu Glu Asn Pro
Ser Ser Leu Phe Asp Leu Val Ala Arg Ile Lys Asn Asn Leu Lys Asn
Ser Pro Asp Leu Tyr Ser His His Phe Gln Ser His Gly Gln Leu Ser
Asp His Pro His Ala Leu Ser Ser Ser Ser Ser His Ala Glu Pro Arg
Gly Glu Asn Ala Val Leu Ser Ser Glu Asp Leu His Lys Pro Gly Gln
Val Ser Val Gln Leu Pro Gly Thr Asn Tyr Val Gly Pro Gly Asn Glu
Leu Gln Ala Gly Pro Pro Gln Ser Ala Val Asp Ser Ala Ala Arg Ile
His Asp Phe Arg Tyr Ser Gln Leu Ala Lys Leu Gly Ile Asn Pro Tyr
Thr His Trp Thr Val Ala Asp Glu Glu Leu Leu Lys Asn Ile Lys Asn
Glu Thr Gly Phe Gln Ala Gln Val Val Lys Asp Tyr Phe Thr Leu Lys
Gly Ala Ala Ala Pro Val Ala His Phe Gln Gly Ser Leu Pro Glu Val
Pro Ala Tyr Asn Ala Ser Glu Lys Tyr Pro Ser Met Thr Ser Val Asn
Ser Ala Glu Ala Ser Thr Gly Ala Gly Gly Gly Gly Ser Asn Ser Val
Lys Ser Met Trp Ser Glu Gly Ala Thr Phe Ser Ala Asn Ser Val Thr
Cys Thr Phe Ser Arg Gln Phe Leu Ile Pro Tyr Asp Pro Glu His His

Tyr Lys Val Phe Ser Pro Ala Ala Ser Ser Cys His Asn Ala Ser Gly
Lys Glu Ala Lys Val Cys Thr Ile Ser Pro Ile Met Gly Tyr Ser Thr
Pro Trp Arg Tyr Leu Asp Phe Asn Ala Leu Asn Leu Phe Phe Ser Pro
Leu Glu Phe Gln His Leu Ile Glu Asn Tyr Gly Ser Ile Ala Pro Asp
Ala Leu Thr Val Thr Ile Ser Glu Ile Ala Val Lys Asp Val Thr Asp
Lys Thr Gly Gly Gly Val Gln Val Thr Asp Ser Thr Thr Gly Arg Leu
Cys Met Leu Val Asp His Glu Tyr Lys Tyr Pro Tyr Val Leu Gly Gln
Gly Gln Asp Thr Leu Ala Pro Glu Leu Pro Ile Trp Val Tyr Phe Pro
Pro Gln Tyr Ala Tyr Leu Thr Val Gly Asp Val Asn Thr Gln Gly Ile
Ser Gly Asp Ser Lys Lys Leu Ala Ser Glu Glu Ser Ala Phe Tyr Val
Leu Glu His Ser Ser Phe Gln Leu Leu Gly Thr Gly Gly Thr Ala Ser
Met Ser Tyr Lys Phe Pro Pro Val Pro Pro Glu Asn Leu Glu Gly Cys
Ser Gln His Phe Tyr Glu Met Tyr Asn Pro Leu Tyr Gly Ser Ser Arg
Val Asp Leu Gln und/oder

```
Met Thr Ile Thr Asn Ser Asp His Met Ser Lys Lys Ser Gly Lys Trp
Trp Glu Ser Asp Asp Lys Phe Ala Lys Ala Val Tyr Gln Gln Phe Val
Glu Phe Tyr Glu Lys Val Thr Gly Thr Asp Leu Glu Leu Ile Gln Ile
Leu Lys Asp His Tyr Asn Ile Ser Leu Asp Asn Pro Leu Glu Asn Pro
Ser Ser Leu Phe Asp Leu Val Ala Arg Ile Lys Asn Asn Leu Lys Asn
Ser Pro Asp Leu Tyr Ser His His Phe Gln Ser His Gly Gln Leu Ser
Asp His Pro His Ala Leu Ser Ser Ser Ser Ser His Ala Glu Pro Arg
Gly Glu Asn Ala Val Leu Ser Ser Glu Asp Leu His Lys Pro Gly Gln
Val Ser Val Gln Leu Pro Gly Thr Asn Tyr Val Gly Pro Gly Asn Glu
Leu Gln Ala Gly Pro Pro Gln Ser Ala Val Asp Ser Ala Ala Arg Ile
His Asp Phe Arg Tyr Ser Gln Leu Ala Lys Leu Gly Ile Asn Pro Tyr
Thr His Trp Thr Val Ala Asp Glu Glu Leu Leu Lys Asn Ile Lys Asn
Glu Thr Gly Phe Gln Ala Gln Val Val Lys Asp Tyr Phe Thr Leu Lys
Gly Ala Ala Ala Pro Val Ala His Phe Gln Gly Ser Leu Pro Glu Val
Pro Ala Tyr Asn Ala Ser Glu Lys Tyr Pro Ser Met Thr Ser Val Asn
Ser Ala Glu Ala Ser Thr Gly Ala Gly Gly Gly Gly Ser Asn Ser Val
Lys Ser Met Trp Ser Glu Gly Ala Thr Phe Ser Ala Asn Ser Val Thr
Cys Thr Phe Ser Arg Gln Phe Leu Ile Pro Tyr Asp Pro Glu His His
Tyr Lys Val Phe Ser Pro Ala Ala Ser Ser Cys His Asn Ala Ser Gly
Lys Glu Ala Lys Val Cys Thr Ile Ser Pro Ile Met Gly Tyr Ser Thr
Pro Trp Arg Tyr Leu Asp Phe Asn Ala Leu Asn Leu Phe Phe Ser Pro

Leu Glu Phe Gln His Leu Ile Glu Asn Tyr Gly Ser Ile Ala Pro Asp
Ala Leu Thr Val Thr Ile Ser Glu Ile Ala Val Lys Asp Val Thr Asp
Lys Thr Gly Gly Gly Val Gln Val Thr Asp Ser Thr Thr Gly Arg Leu
Cys Ser Asn
```

oder Teilsequenzen davon aufweist die zum Nachweis von Antikörpern gegen Parvovirus B19 in den Untersuchungsflüssigkeitengeeignet sind.

2.  Immunologisch aktives Peptid oder Polypeptid nach Anspruch 1, dadurch gekennzeichnet, daß es als Fusionsprotein vorliegt, wobei dieses Fusionsprotein wenigstens einen Teil der β-Galactosidase oder der Glutathion-S-Transferase aufweist.

3.  Testkit zum Nachweis von Antikörpern gegen das humane Parvovirus B19, dadurch gekennzeichnet, daß es wenigstens ein immunologisch aktives Peptid oder Polypeptid nach Anspruch 1 aufweist, das mit den in den Untersuchungsflüssigkeiten vorhandenen Antikörpern reagieren kann und, daß es wenigstens eine Anzeigekomponente aufweist, die den Nachweis von Komplexen aus immunologisch aktivem Peptid und Antikörper ermöglicht.

4.  Testkit nach Anspruch 3, dadurch gekennzeichnet, daß die Anzeigekomponente ein gegen den nachzuweisenden Antikörper gerichteter Antikörper ist, der eine Markierung aufweist.

5.  Testkit nach Anspruch 4 , dadurch gekennzeichnet, daß die Markierung in einem radioaktiven Isotop besteht.

6.  Testkit nach Anspruch 4 , dadurch gekennzeichnet, daß die Markierung aus einem Enzym besteht, das eine Farbreaktion katalysieren kann.

7. Testkit nach Anspruch 4 , dadurch gekennzeichnet, daß das immunologisch aktive Peptid oder Polypeptid biotinyliert ist und die Anzeigekomponente Avidin oder Streptavidin mit daran kovalent gebundenem Enyzm, insbesondere Peroxidase, ist.

8. Testkit nach einem der Ansprüche 4 bis 7 , dadurch gekennzeichnet, daß es ein ELISA-Kit ist.

9. Testkit nach Anspruch 8 , dadurch gekennzeichnet, daß wenigstens ein immunologisch aktives Peptid oder Polypeptid nach Anspruch 1 an Mikrotiterplatten gekoppelt ist und, daß die Anzeigekomponente aus anti-human IgG- und/oder IgM-Antikörpern besteht, an die ein eine Farbreaktion katalysierendes Enzym gekoppelt ist.

10. Testkit nach Anspruch 8 , dadurch gekennzeichnet, daß monoklonale Antikörper gegen Human-IgM-Antikörper an Mikrotiterplatten gekoppelt sind und, daß die Anzeigebomponente ein biotinyliertes immunologisch aktives Peptid oder Polypeptid nach Anspruch 1 ist, das mit Avidin oder streptavidin mit daran kovalent gebundenem Enzym zusammenwirkt.

11. Verfahren zur Reinigung von immunologisch aktiven Peptiden oder Polypeptiden nach Anspruch 1 , dadurch gekennzeichnet, daß es eine Abtrennung unlöslicher Bestandteile, eine Auftrennung durch eine DE-AE-Sephazall-Säule und eine weitere Reinigung mittels einer Anionenaustauscher-Säule im HPLC in 8M Harnstoff umfaßt.

12. Verfahren nach Anspruch 11, dadurch gekennzeichnet, daß es außerdem eine Affinitäts-Chromatographie umfaßt.

13. Verfahren nach Anspruch 12, dadurch gekennzeichnet, daß die Affinitäts-Chromatographie mit einer Glutathion-gekoppelten Gel-Matrix erfolgt.

14. Verwendung von wenigstens einer DNA-Sequenz ausgewählt unter

O-1:
GTG AAT TCT GAT CAT ATG AGT AAA AAA AGT GGC AAA TGG,

O-2:
C TTC GGT CGT GAC CAC GTC CTC CCC

O-3:
G AGG AAT TCT CTG ATC ATG ACT TCA GTT AAT TCT GCA GAA GCC

O-4:
GAG GGG TGG CAC GGG AGT CGG TCC TTC GAA GAG

O-5:
G CTA CAA GCT GGG CCC CCG CAA AG

zum direkten Erregernachweis mittels DNA-Amplifizierung, insbesondere mittels Polymerase-chain-reaction.

15. Verwendung von immunologisch aktiven Peptiden nach Anspruch 1 zur Herstellung eines Impfstoffs gegen Infektionen mit Parvovirus B19.

**Claims**

1. Immunologically active peptide or polypeptide which has a part of the amino-acid sequence of the capsid proteins VP1 or VP2 of parvovirus B19, characterized in that it is free of impurities which may interfere with the detection of parvovirus B19 specific antibodies, and the polypeptide which is a partial sequence of 8 to 50 amino-acid residues, particularly 10 to 32 amino-acid residues of the peptide PAN1 as depicted in Fig. 2-1, or has the amino-acid sequence

Asn Pro Tyr Thr His Trp Thr Val Ala Asp Glu Glu Leu Leu Lys His Ile Lys and/or

Ser Lys Lys Ser Gly Lys Trp Trp Glu Ser Asp Asp Lys Phe Ala Lys Ala Val Tyr and/or

Leu Lys Asp His Tyr Asn Ile Ser Leu Asp Asn Pro Leu Glu Asn Pro Ser Ser and/or

Ile Lys Asn Asn Leu Lys Asn Ser Pro Asp Leu Tyr Ser His His Phe Gln Ser His Gly Gln Leu Ser Asp His Pro His Ala and/or

Ser Ser His Ala Glu Pro Arg Gly Glu Asn Ala Val Leu Ser Ser Glu Asp Leu His Lys Pro Gly Gln Val and/or

Asn Tyr Val Gly Pro Gly Asn Glu Leu Gln Ala Gly Pro Pro Gln Ser Ala Val Asp Ser Ala Ala Arg Ile His Asp Phe Arg Tyr Ser Gln Leu

and/or

Pro Tyr Thr His Trp Thr Val Ala Asp Glu Glu Leu Leu Lys Asn Ile Lys Asn Glu Thr Gly Phe and/or

Asn Ala Ser Glu Lys Tyr Pro Ser Met Thr Ser Val Asn Ser Ala Glu Ala Ser

and/or the amino-acid sequence

His Met Ser Lys Lys Ser Gly Lys Trp Trp Glu Ser Asp Asp Lys Phe
Ala Lys Ala Val Tyr Gln Gln Phe Val Glu Phe Tyr Glu Lys Val Thr
Gly Thr Asp Leu Glu Leu Ile Gln Ile Leu Lys Asp His Tyr Asn Ile
Ser Leu Asp Asn Pro Leu Glu Asn Pro Ser Ser Leu Phe Asp Leu Val
Ala Arg Ile Lys Asn Asn Leu Lys Asn Ser Pro Asp Leu Tyr Ser His
His Phe Gln Ser His Gly Gln Leu Ser Asp His Pro His Ala Leu Ser
Ser Ser Ser Ser His Ala Glu Pro Arg Gly Glu Asn Ala Val Leu Ser
Ser Glu Asp Leu His Lys Pro Gly Gln Val Ser Val Gln Leu Pro Gly
Thr Asn Tyr Val Gly Pro Gly Asn Glu Leu Gln Ala Gly Pro Pro Gln
Ser Ala Val Asp Ser Ala Ala Arg Ile His Asp Phe Arg Tyr Ser Gln
Leu Ala Lys Leu Gly Ile Asn Pro Tyr Thr His Trp Thr Val Ala Asp
Glu Glu Leu Leu Lys Asn Ile Lys Asn Glu Thr Gly Phe Gln Ala Gln
Val Val Lys Asp Tyr Phe Thr Leu Lys Gly Ala Gly Glu Phe Ile Val
Thr Asp and/or

Gly Ser Arg Arg Pro Asp His Met Ser Lys Lys Ser Gly Lys Trp Trp
Glu Ser Asp Asp Lys Phe Ala Lys Ala Val Tyr Gln Gln Phe Val Glu
Phe Tyr Glu Lys Val Thr Gly Thr Asp Leu Glu Leu Ile Gln Ile Leu
Lys Asp His Tyr Asn Ile Ser Leu Asp Asn Pro Leu Glu Asn Pro Ser
Ser Leu Phe Asp Leu Val Ala Arg Ile Lys Asn Asn Leu Lys Asn Ser
Pro Asp Leu Tyr Ser His His Phe Gln Ser His Gly Gln Leu Ser Asp
His Pro His Ala Leu Ser Ser Ser Ser Ser His Ala Glu Pro Arg Gly
Glu Asn Ala Val Leu Ser Ser Glu Asp Leu His Lys Pro Gly Gln Val
Ser Val Gln Leu Pro Gly Thr Asn Tyr Val Gly Pro Gly Asn Glu Leu

Gln Ala Gly Pro Pro Gln Ser Ala Val Gly Asp Pro Arg Glu Phe Ile
Val Thr Asp and/or

Gly Ile Leu Ser Arg Arg Pro Asp His Met Ser Lys Lys Ser Gly Lys
Trp Trp Glu Ser Asp Asp Lys Phe Ala Lys Ala Val Tyr Gln Gln Phe
Val Glu Phe Tyr Glu Lys Val Thr Gly Thr Asp Leu Glu Leu Ile Gln
Ile Leu Lys Asp His Tyr Asn Ile Ser Leu Asp Asn Pro Leu Glu Asn
Pro Ser Ser Leu Phe Asp Leu Val Ala Arg Ile Lys Asn Asn Leu Lys
Asn Ser Pro Asp Leu Tyr Ser His His Phe Gln Ser His Gly Gln Leu
Ser Asp His Pro His Ala Leu Ser Ser Ser Ser Ser His Ala Glu Pro
Arg Gly Glu Asn Ala Val Leu Ser Ser Glu Asp Leu His Lys Pro Gly
Gln Val Ser Val Gln Leu Pro Gly Thr Asn Tyr Val Gly Pro Gly Asn
Glu Leu Gln Ala Gly Pro Pro Gln Ser Ala Val Gly Asp Pro Leu Glu
Asp Pro Arg Val Pro Ser Ser Asn Ser and/or

28

```
Gly Ser Arg Arg Pro Asp His Met Ser Lys Lys Ser Gly Lys Trp Trp
Glu Ser Asp Asp Lys Phe Ala Lys Ala Val Tyr Gln Gln Phe Val Glu
Phe Tyr Glu Lys Val Thr Gly Thr Asp Leu Glu Leu Ile Gln Ile Leu
Lys Asp His Tyr Asn Ile Ser Leu Asp Asn Pro Leu Glu Asn Pro Ser
Ser Leu Phe Asp Leu Val Ala Arg Ile Lys Asn Asn Leu Lys Asn Ser
Pro Asp Leu Tyr Ser His His Phe Gln Ser His Gly Gln Leu Ser Asp
His Pro His Ala Leu Ser Ser Ser Ser Ser His Ala Glu Pro Arg Gly
Glu Asn Ala Val Leu Ser Ser Glu Asp Leu His Lys Pro Gly Gln Val
Ser Val Gln Leu Pro Gly Thr Asn Tyr Val Gly Pro Gly Asn Glu Leu
Gln Ala Gly Pro Pro Gln Ser Ala Val Asp Ser Ala Ala Arg Ile His
Asp Phe Arg Tyr Ser Gln Leu Ala Lys Leu Gly Ile Asn Pro Tyr Thr
His Trp Thr Val Ala Asp Glu Glu Leu Leu Lys Asn Ile Lys Asn Glu
Thr Gly Phe Gln Ala Gln Val Val Lys Asp Tyr Phe Thr Leu Lys Gly
Ala Ala Ala Pro Val Ala His Phe Gln Gly Ser Leu Pro Glu Val Pro
Ala Tyr Asn Ala Ser Glu Lys Tyr Pro Ser Met Thr Ser Val Asn Ser
Ala Gly Arg Arg Ile Pro Gly Asn Ser Ser  and/or
```

```
Met Thr Met Ile Thr Pro Ser Leu His Ala Cys Met Leu Val Asp His
Glu Tyr Lys Tyr Pro Tyr Val Leu Gly Gln Gly Gln Asp Thr Leu Ala
Pro Glu Leu Pro Ile Trp Val Tyr Phe Pro Pro Gln Tyr Ala Tyr Leu
Thr Val Gly Asp Val Asn Thr Gln Gly Ile Ser Gly Asp Ser Lys Lys
Leu Ala Ser Glu Glu Ser Ala Phe Tyr Val Leu Glu His Ser Ser Phe
Gln Leu Leu Gly Thr Gly Gly Thr Ala Ser Met Ser Tyr Lys Phe Pro
Pro Val Pro Pro Glu Asn Leu Glu Gly Cys Ser Gln His Phe Tyr Glu
Met Tyr Asn Pro Leu Tyr Gly Ser Arg Leu Gly Val Pro Asp Thr Leu
Gly Gly Asp Pro Lys Phe Arg Ser Leu Thr His Glu Asp His Ala Ile
Gln Pro Gln Asn Phe Met Pro Gly Pro Leu Val Asn Ser Val Ser Thr
Lys Glu Gly Asp Ser Ser Asn Thr Gly Ala Gly Lys Ala Leu Thr Gly
Leu Ser Thr Gly Thr Ser Gln Asn Thr Arg Ile Ser Leu Arg Pro Gly
Pro Val Ser Gln Pro Tyr His His Trp Asp Thr Asp Lys Tyr Val Thr
Gly Ile Asn Ala Ile Ser His Gly Gln Thr Thr Tyr Gly Asn Ala Glu
Asp Lys Glu Tyr Gln Gln Gly Val Gly Arg Phe Pro Asn Glu Lys Glu
Gln Leu Lys Gln Leu Gln Gly Leu Asn Met His Thr Tyr Phe Pro Asn
Lys Gly Thr Gln Gln Tyr Thr Asp Gln Ile Glu Arg Pro Leu Met Val
Gly Ser Val Trp Asn Arg Arg Ala Leu His Tyr Glu Ser Gln Leu Trp
Ser Lys Ile Pro Asn Leu Asp Asp Ser Phe Lys Thr Gln Phe Ala Ala
Leu Gly Gly Trp Gly Leu His Gln Pro Pro Pro Gln Ile Phe Leu Lys
Gln Tyr Ala Val Gly Ile Met Thr Val Thr Met Thr Phe Lys Leu Gly
Pro Arg Lys Ala Thr Gly Arg Trp Asn Pro Gln Pro Gly Val Tyr Pro
Pro His Ala Ala Gly His Leu Pro Tyr Val Leu Tyr Asp Pro Thr Ala
Thr Asp Ala Lys Gln His His Arg His Gly Tyr Glu Lys Pro Glu Glu
Leu Trp Thr Ala Lys Ser Arg Val His Pro Leu and/or
```

Met Thr Met Ile Thr Pro Ser Leu Ala Ala Glu Ala Ser Thr Gly Ala
Gly Gly Gly Gly Ser Asn Ser Val Lys Ser Met Trp Ser Glu Gly Ala
Thr Phe Ser Ala Asn Ser Val Thr Cys Thr Phe Ser Arg Gln Phe Leu
Ile Pro Tyr Asp Pro Glu His His Tyr Lys Val Phe Ser Pro Ala Ala
Ser Ser Cys His Asn Ala Ser Gly Lys Glu Ala Lys Val Cys Thr Ile
Ser Pro Ile Met Gly Tyr Ser Thr Pro Trp Arg Tyr Leu Asp Phe Asn
Ala Leu Asn Leu Phe Phe Ser Pro Leu Glu Phe Gln His Leu Ile Glu
Asn Tyr Gly Ser Ile Ala Pro Asp Ala Leu Thr Val Thr Ile Ser Glu
Ile Ala Val Lys Asp Val Thr Asp Lys Thr Gly Gly Gly Val Gln Val
Thr Asp Ser Thr Thr Gly Arg Leu Cys Met Leu Val Asp His Glu Tyr
Lys Tyr Pro Tyr Val Leu Gly Gln Gly Gln Asp Thr Leu Ala Pro Glu
Leu Pro Ile Trp Val Tyr Phe Pro Pro Gln Tyr Ala Tyr Leu Thr Val
Gly Asp Val Asn Thr Gln Gly Ile Ser Gly Asp Ser Lys Lys Leu Ala
Ser Glu Glu Ser Ala Phe Tyr Val Leu Glu His Ser Ser Phe Gln Leu
Leu Gly Thr Gly Gly Thr Ala Ser Met Ser Tyr Lys Phe Pro Pro Val
Pro Pro Glu Asn Leu Glu Gly Cys Arg Ser Thr Asp Pro Arg Glu Phe
Thr Gly Arg Arg Phe Thr Thr Ser and/or

Met Ser Lys Lys Ser Gly Lys Trp Trp Glu Ser Asp Asp Lys Phe Ala
Lys Ala Val Tyr Gln Gln Phe Val Glu Phe Tyr Glu Lys Val Thr Gly
Thr Asp Leu Glu Leu Ile Gln Ile Leu Lys Asp His Tyr Asn Ile Ser
Leu Asp Asn Pro Leu Glu Asn Pro Ser Ser Leu Phe Asp Leu Val Ala
Arg Ile Lys Asn Asn Leu Lys Asn Ser Pro Asp Leu Tyr Ser His His
Phe Gln Ser His Gly Gln Leu Ser Asp His Pro His Ala Leu Ser Ser
Ser Ser Ser His Ala Glu Pro Arg Gly Glu Asn Ala Val Leu Ser Ser
Glu Asp Leu His Lys Pro Gly Gln Val Ser Val Gln Leu Pro Gly Thr
Asn Tyr Val Gly Pro Gly Asn Glu Leu Gln Ala Gly Pro Pro Gln Ser
Ala Val Asp Ser Ala Ala Arg Ile His Asp Phe Arg Tyr Ser Gln Leu
Ala Lys Leu Gly Ile Asn Pro Tyr Thr His Trp Thr Val Ala Asp Glu
Glu Leu Leu Lys Asn Ile Lys Asn Glu Thr Gly Phe Gln Ala Gln Val
Val Lys Asp Tyr Phe Thr Leu Lys Gly Ala Ala Ala Pro Val Ala His
Phe Gln Gly Ser Leu Pro Glu Val Pro Ala Tyr Asn Ala Ser Glu Lys
Tyr Pro Ser and/or

31

```
Met Thr Ile Thr Asn Ser Asp His Met Ser Lys Lys Ser Gly Lys Trp
Trp Glu Ser Asp Asp Lys Phe Ala Lys Ala Val Tyr Gln Gln Phe Val
Glu Phe Tyr Glu Lys Val Thr Gly Thr Asp Leu Glu Leu Ile Gln Ile
Leu Lys Asp His Tyr Asn Ile Ser Leu Asp Asn Pro Leu Glu Asn Pro
Ser Ser Leu Phe Asp Leu Val Ala Arg Ile Lys Asn Asn Leu Lys Asn
Ser Pro Asp Leu Tyr Ser His His Phe Gln Ser His Gly Gln Leu Ser
Asp His Pro His Ala Leu Ser Ser Ser Ser Ser His Ala Glu Pro Arg
Gly Glu Asn Ala Val Leu Ser Ser Glu Asp Leu His Lys Pro Gly Gln
Val Ser Val Gln Leu Pro Gly Thr Asn Tyr Val Gly Pro Gly Asn Glu
Leu Gln Ala Gly Pro Pro Gln Ser Ala Val Asp Ser Ala Ala Arg Ile
His Asp Phe Arg Tyr Ser Gln Leu Ala Lys Leu Gly Ile Asn Pro Tyr
Thr His Trp Thr Val Ala Asp Glu Glu Leu Leu Lys Asn Ile Lys Asn
Glu Thr Gly Phe Gln Ala Gln Val Val Lys Asp Tyr Phe Thr Leu Lys
Gly Ala Ala Ala Pro Val Ala His Phe Gln Gly Ser Leu Pro Glu Val
Pro Ala Tyr Asn Ala Ser Glu Lys Tyr Pro Ser Met Thr Ser Val Asn
Ser Ala Glu Ala Ser Thr Gly Ala Gly Gly Gly Gly Ser Asn Ser Val
Lys Ser Met Trp Ser Glu Gly Ala Thr Phe Ser Ala Asn Ser Val Thr
Cys Thr Phe Ser Arg Gln Phe Leu Ile Pro Tyr Asp Pro Glu His His
Tyr Lys Val Phe Ser Pro Ala Ala Ser Ser Cys His Asn Ala Ser Gly
Lys Glu Ala Lys Val Cys Thr Ile Ser Pro Ile Met Gly Tyr Ser Thr
Pro Trp Arg Tyr Leu Asp Phe Asn Ala Leu Asn Leu Phe Phe Ser Pro
Leu Glu Phe Gln His Leu Ile Glu Asn Tyr Gly Ser Ile Ala Pro Asp
Ala Leu Thr Val Thr Ile Ser Glu Ile Ala Val Lys Asp Val Thr Asp
Lys Thr Gly Gly Gly Val Gln Val Thr Asp Ser Thr Thr Gly Arg Leu
Cys Met Leu Val Asp His Glu Tyr Lys Tyr Pro Tyr Val Leu Gly Gln
Gly Gln Asp Thr Leu Ala Pro Glu Leu Pro Ile Trp Val Tyr Phe Pro
Pro Gln Tyr Ala Tyr Leu Thr Val Gly Asp Val Asn Thr Gln Gly Ile
Ser Gly Asp Ser Lys Lys Leu Ala Ser Glu Glu Ser Ala Phe Tyr Val
Leu Glu His Ser Ser Phe Gln Leu Leu Gly Thr Gly Gly Thr Ala Ser
Met Ser Tyr Lys Phe Pro Pro Val Pro Pro Glu Asn Leu Glu Gly Cys
Ser Gln His Phe Tyr Glu Met Tyr Asn Pro Leu Tyr Gly Ser Ser Arg
Val Asp Leu Gln and/or
```

```
Met Thr Ile Thr Asn Ser Asp His Met Ser Lys Lys Ser Gly Lys Trp
Trp Glu Ser Asp Asp Lys Phe Ala Lys Ala Val Tyr Gln Gln Phe Val
Glu Phe Tyr Glu Lys Val Thr Gly Thr Asp Leu Glu Leu Ile Gln Ile
Leu Lys Asp His Tyr Asn Ile Ser Leu Asp Asn Pro Leu Glu Asn Pro
Ser Ser Leu Phe Asp Leu Val Ala Arg Ile Lys Asn Asn Leu Lys Asn
Ser Pro Asp Leu Tyr Ser His His Phe Gln Ser His Gly Gln Leu Ser
Asp His Pro His Ala Leu Ser Ser Ser Ser Ser His Ala Glu Pro Arg
Gly Glu Asn Ala Val Leu Ser Ser Glu Asp Leu His Lys Pro Gly Gln
Val Ser Val Gln Leu Pro Gly Thr Asn Tyr Val Gly Pro Gly Asn Glu
Leu Gln Ala Gly Pro Pro Gln Ser Ala Val Asp Ser Ala Ala Arg Ile
His Asp Phe Arg Tyr Ser Gln Leu Ala Lys Leu Gly Ile Asn Pro Tyr
Thr His Trp Thr Val Ala Asp Glu Glu Leu Leu Lys Asn Ile Lys Asn
Glu Thr Gly Phe Gln Ala Gln Val Val Lys Asp Tyr Phe Thr Leu Lys
Gly Ala Ala Ala Pro Val Ala His Phe Gln Gly Ser Leu Pro Glu Val
Pro Ala Tyr Asn Ala Ser Glu Lys Tyr Pro Ser Met Thr Ser Val Asn
Ser Ala Glu Ala Ser Thr Gly Ala Gly Gly Gly Gly Ser Asn Ser Val
Lys Ser Met Trp Ser Glu Gly Ala Thr Phe Ser Ala Asn Ser Val Thr
Cys Thr Phe Ser Arg Gln Phe Leu Ile Pro Tyr Asp Pro Glu His His
Tyr Lys Val Phe Ser Pro Ala Ala Ser Ser Cys His Asn Ala Ser Gly
Lys Glu Ala Lys Val Cys Thr Ile Ser Pro Ile Met Gly Tyr Ser Thr
Pro Trp Arg Tyr Leu Asp Phe Asn Ala Leu Asn Leu Phe Phe Ser Pro

Leu Glu Phe Gln His Leu Ile Glu Asn Tyr Gly Ser Ile Ala Pro Asp
Ala Leu Thr Val Thr Ile Ser Glu Ile Ala Val Lys Asp Val Thr Asp
Lys Thr Gly Gly Gly Val Gln Val Thr Asp Ser Thr Thr Gly Arg Leu
Cys Ser Asn
```

or partial sequences thereof which are suitable for the detection of antibodies against parvovirus B19 in the liquid samples.

2.  Immunologically active peptide or polypeptide according to claim 1, characterized in that it is in the form of a fusion protein, where this fusion protein has at least a part of β-galactosidase or of glutathione S-transferase.

3.  Test kit for the detection of antibodies against human parvovirus B19, characterized in that it has at least one immunologically active peptide or polypeptide according to claim 1, which is able to react with the antibodies present in the liquid samples, and in that it has at least one indicator component which makes it possible to detect complexes of immunologically active peptide and antibody.

4.  Test kit according to claim 3, characterized in that the indicator component is an antibody which is directed against the antibody to be detected and has a label.

5.  Test kit according to claim 4, characterized in that the label consists of a radioactive isotope.

6.  Test kit according to claim 4, characterized in that the label consists of an enzyme which is able to catalyze a colour reaction.

7.  Test kit according to claim 4, characterized in that the immunologically active peptide or polypeptide is biotinylated, and the indicator component is avidin or streptavidin with an enzyme, especially peroxidase, covalently bonded thereto.

8. Test kit according to any of claims 4 to 7, characterized in that it is an ELISA kit.

9. Test kit according to claim 8, characterized in that at least one immunologically active peptide or polypeptide according to claim 1 is coupled to microtitre plates, and in that the indicator component consists of anti-human IgG and/or IgM antibodies to which an enzyme catalyzing a colour reaction is coupled.

10. Test kit according to claim 8, characterized in that monoclonal antibodies against human IgM antibodies are coupled to microtitre plates, and in that the indicator component is a biotinylated immunologically active peptide or polypeptide according to claim 1, which cooperates with avidin or streptavidin with an enzyme covalently bonded thereto.

11. Process for the purification of immunologically active peptides or polypeptides according to claim 1, characterized in that it comprises a removal of insoluble constituents, a fractionation by a DEAE-sephazell column and another purification by means of an anion exchanger column in HPLC in 8M urea.

12. Process according to claim 11, characterized in that it additionally comprises an affinity chromatography.

13. Process according to claim 12, characterized in that the affinity chromatography is carried out with a glutathione-coupled gel matrix.

14. Use of at least one DNA sequence selected from

O-1:
GTG AAT TCT GAT CAT ATG AGT AAA AAA AGT GGC AAA TGG,

O-2:
C TTC GGT CGT GAC CAC GTC CTC CCC

O-3:
G AGG AAT TCT CTG ATC ATG ACT TCA GTT AAT TCT GCA GAA GCC

O-4:
GAG GGG TGG CAC GGG AGT CGG TCC TTC GAA GAG

O-5:
G CTA CAA GCT GGG CCC CCG CAA AG

for the direct detection of pathogen by means of DNA amplification, especially by means of polymerase chain reaction.

15. Use of immunologically active peptides according to claim 1 for the preparation of a vaccine against infections with parvovirus B19.

**Revendications**

1. Peptide ou polypeptide immunologiquement actif, qui présente une partie de la séquence d'acides aminés des protéines de capside VP1 ou VP2 du parvovirus B19, caractérisé en ce qu'il est exempt d'impuretés, qui peuvent entraver la caractérisation d'anticorps spécifiques du parvovirus B19, et qui présente le polypeptide qui est une séquence partielle de 8 à 50 restes d'acides aminés, en particulier de 10 à 32 restes d'acides aminés du peptide PAN1, tel que représenté dans l'illustration 2-1, ou la sequence d'acides ami-

nés

Asn Pro Tyr Thr His Trp Thr Val Ala Asp Glu Glu Leu Leu Lys His
Ile Lys et/ou

Ser Lys Lys Ser Gly Lys Trp Trp Glu Ser Asp Asp Lys Phe Ala Lys
Ala Val Tyr et/ou

Leu Lys Asp His Tyr Asn Ile Ser Leu Asp Asn Pro Leu Glu Asn Pro
Ser Ser et/ou

Ile Lys Asn Asn Leu Lys Asn Ser Pro Asp Leu Tyr Ser His His Phe
Gln Ser His Gly Gln Leu Ser Asp His Pro His Ala et/ou

Ser Ser His Ala Glu Pro Arg Gly Glu Asn Ala Val Leu Ser Ser Glu
Asp Leu His Lys Pro Gly Gln Val et/ou ,

Asn Tyr Val Gly Pro Gly Asn Glu Leu Gln Ala Gly Pro Pro Gln Ser
Ala Val Asp Ser Ala Ala Arg Ile His Asp Phe Arg Tyr Ser Gln Leu

et/ou

Pro Tyr Thr His Trp Thr Val Ala Asp Glu Glu Leu Leu Lys Asn Ile
Lys Asn Glu Thr Gly Phe et/ou

Asn Ala Ser Glu Lys Tyr Pro Ser Met Thr Ser Val Asn Ser Ala Glu
Ala Ser

et/ou qui présente la séquence d'acides aminés

His Met Ser Lys Lys Ser Gly Lys Trp Trp Glu Ser Asp Asp Lys Phe
Ala Lys Ala Val Tyr Gln Gln Phe Val Glu Phe Tyr Glu Lys Val Thr
Gly Thr Asp Leu Glu Leu Ile Gln Ile Leu Lys Asp His Tyr Asn Ile
Ser Leu Asp Asn Pro Leu Glu Asn Pro Ser Ser Leu Phe Asp Leu Val
Ala Arg Ile Lys Asn Asn Leu Lys Asn Ser Pro Asp Leu Tyr Ser His
His Phe Gln Ser His Gly Gln Leu Ser Asp His Pro His Ala Leu Ser
Ser Ser Ser Ser His Ala Glu Pro Arg Gly Glu Asn Ala Val Leu Ser
Ser Glu Asp Leu His Lys Pro Gly Gln Val Ser Val Gln Leu Pro Gly
Thr Asn Tyr Val Gly Pro Gly Asn Glu Leu Gln Ala Gly Pro Pro Gln
Ser Ala Val Asp Ser Ala Ala Arg Ile His Asp Phe Arg Tyr Ser Gln
Leu Ala Lys Leu Gly Ile Asn Pro Tyr Thr His Trp Thr Val Ala Asp
Glu Glu Leu Leu Lys Asn Ile Lys Asn Glu Thr Gly Phe Gln Ala Gln
Val Val Lys Asp Tyr Phe Thr Leu Lys Gly Ala Gly Glu Phe Ile Val
Thr Asp et/ou

Gly Ser Arg Arg Pro Asp His Met Ser Lys Lys Ser Gly Lys Trp Trp
Glu Ser Asp Asp Lys Phe Ala Lys Ala Val Tyr Gln Gln Phe Val Glu
Phe Tyr Glu Lys Val Thr Gly Thr Asp Leu Glu Leu Ile Gln Ile Leu
Lys Asp His Tyr Asn Ile Ser Leu Asp Asn Pro Leu Glu Asn Pro Ser
Ser Leu Phe Asp Leu Val Ala Arg Ile Lys Asn Asn Leu Lys Asn Ser
Pro Asp Leu Tyr Ser His His Phe Gln Ser His Gly Gln Leu Ser Asp
His Pro His Ala Leu Ser Ser Ser Ser Ser His Ala Glu Pro Arg Gly
Glu Asn Ala Val Leu Ser Ser Glu Asp Leu His Lys Pro Gly Gln Val
Ser Val Gln Leu Pro Gly Thr Asn Tyr Val Gly Pro Gly Asn Glu Leu

Gln Ala Gly Pro Pro Gln Ser Ala Val Gly Asp Pro Arg Glu Phe Ile
Val Thr Asp et/ou

36

EP 0 514 413 B1

```
Gly Ile Leu Ser Arg Arg Pro Asp His Met Ser Lys Lys Ser Gly Lys
Trp Trp Glu Ser Asp Asp Lys Phe Ala Lys Ala Val Tyr Gln Gln Phe
Val Glu Phe Tyr Glu Lys Val Thr Gly Thr Asp Leu Glu Leu Ile Gln
Ile Leu Lys Asp His Tyr Asn Ile Ser Leu Asp Asn Pro Leu Glu Asn
Pro Ser Ser Leu Phe Asp Leu Val Ala Arg Ile Lys Asn Asn Leu Lys
Asn Ser Pro Asp Leu Tyr Ser His His Phe Gln Ser His Gly Gln Leu
Ser Asp His Pro His Ala Leu Ser Ser Ser Ser Ser His Ala Glu Pro
Arg Gly Glu Asn Ala Val Leu Ser Ser Glu Asp Leu His Lys Pro Gly
Gln Val Ser Val Gln Leu Pro Gly Thr Asn Tyr Val Gly Pro Gly Asn
Glu Leu Gln Ala Gly Pro Pro Gln Ser Ala Val Gly Asp Pro Leu Glu
Asp Pro Arg Val Pro Ser Ser Asn Ser et/ou
```

```
Gly Ser Arg Arg Pro Asp His Met Ser Lys Lys Ser Gly Lys Trp Trp
Glu Ser Asp Asp Lys Phe Ala Lys Ala Val Tyr Gln Gln Phe Val Glu
Phe Tyr Glu Lys Val Thr Gly Thr Asp Leu Glu Leu Ile Gln Ile Leu
Lys Asp His Tyr Asn Ile Ser Leu Asp Asn Pro Leu Glu Asn Pro Ser
Ser Leu Phe Asp Leu Val Ala Arg Ile Lys Asn Asn Leu Lys Asn Ser
Pro Asp Leu Tyr Ser His His Phe Gln Ser His Gly Gln Leu Ser Asp
His Pro His Ala Leu Ser Ser Ser Ser Ser His Ala Glu Pro Arg Gly
Glu Asn Ala Val Leu Ser Ser Glu Asp Leu His Lys Pro Gly Gln Val
Ser Val Gln Leu Pro Gly Thr Asn Tyr Val Gly Pro Gly Asn Glu Leu
Gln Ala Gly Pro Pro Gln Ser Ala Val Asp Ser Ala Ala Arg Ile His
Asp Phe Arg Tyr Ser Gln Leu Ala Lys Leu Gly Ile Asn Pro Tyr Thr
His Trp Thr Val Ala Asp Glu Glu Leu Leu Lys Asn Ile Lys Asn Glu
Thr Gly Phe Gln Ala Gln Val Val Lys Asp Tyr Phe Thr Leu Lys Gly
Ala Ala Ala Pro Val Ala His Phe Gln Gly Ser Leu Pro Glu Val Pro
Ala Tyr Asn Ala Ser Glu Lys Tyr Pro Ser Met Thr Ser Val Asn Ser
Ala Gly Arg Arg Ile Pro Gly Asn Ser Ser et/ou
```

37

```
Met Thr Met Ile Thr Pro Ser Leu His Ala Cys Met Leu Val Asp His
Glu Tyr Lys Tyr Pro Tyr Val Leu Gly Gln Gly Gln Asp Thr Leu Ala
Pro Glu Leu Pro Ile Trp Val Tyr Phe Pro Pro Gln Tyr Ala Tyr Leu
Thr Val Gly Asp Val Asn Thr Gln Gly Ile Ser Gly Asp Ser Lys Lys
Leu Ala Ser Glu Glu Ser Ala Phe Tyr Val Leu Glu His Ser Ser Phe
Gln Leu Leu Gly Thr Gly Gly Thr Ala Ser Met Ser Tyr Lys Phe Pro
Pro Val Pro Pro Glu Asn Leu Glu Gly Cys Ser Gln His Phe Tyr Glu
Met Tyr Asn Pro Leu Tyr Gly Ser Arg Leu Gly Val Pro Asp Thr Leu
Gly Gly Asp Pro Lys Phe Arg Ser Leu Thr His Glu Asp His Ala Ile
Gln Pro Gln Asn Phe Met Pro Gly Pro Leu Val Asn Ser Val Ser Thr
Lys Glu Gly Asp Ser Ser Asn Thr Gly Ala Gly Lys Ala Leu Thr Gly
Leu Ser Thr Gly Thr Ser Gln Asn Thr Arg Ile Ser Leu Arg Pro Gly
Pro Val Ser Gln Pro Tyr His His Trp Asp Thr Asp Lys Tyr Val Thr
Gly Ile Asn Ala Ile Ser His Gly Gln Thr Thr Tyr Gly Asn Ala Glu
Asp Lys Glu Tyr Gln Gln Gly Val Gly Arg Phe Pro Asn Glu Lys Glu
Gln Leu Lys Gln Leu Gln Gly Leu Asn Met His Thr Tyr Phe Pro Asn
Lys Gly Thr Gln Gln Tyr Thr Asp Gln Ile Glu Arg Pro Leu Met Val
Gly Ser Val Trp Asn Arg Arg Ala Leu His Tyr Glu Ser Gln Leu Trp
Ser Lys Ile Pro Asn Leu Asp Asp Ser Phe Lys Thr Gln Phe Ala Ala
Leu Gly Gly Trp Gly Leu His Gln Pro Pro Pro Gln Ile Phe Leu Lys
Gln Tyr Ala Val Gly Ile Met Thr Val Thr Met Thr Phe Lys Leu Gly
Pro Arg Lys Ala Thr Gly Arg Trp Asn Pro Gln Pro Gly Val Tyr Pro
Pro His Ala Ala Gly His Leu Pro Tyr Val Leu Tyr Asp Pro Thr Ala
Thr Asp Ala Lys Gln His His Arg His Gly Tyr Glu Lys Pro Glu Glu
Leu Trp Thr Ala Lys Ser Arg Val His Pro Leu et/ou
```

Met Thr Met Ile Thr Pro Ser Leu Ala Ala Glu Ala Ser Thr Gly Ala
Gly Gly Gly Gly Ser Asn Ser Val Lys Ser Met Trp Ser Glu Gly Ala
Thr Phe Ser Ala Asn Ser Val Thr Cys Thr Phe Ser Arg Gln Phe Leu
Ile Pro Tyr Asp Pro Glu His His Tyr Lys Val Phe Ser Pro Ala Ala
Ser Ser Cys His Asn Ala Ser Gly Lys Glu Ala Lys Val Cys Thr Ile
Ser Pro Ile Met Gly Tyr Ser Thr Pro Trp Arg Tyr Leu Asp Phe Asn
Ala Leu Asn Leu Phe Phe Ser Pro Leu Glu Phe Gln His Leu Ile Glu
Asn Tyr Gly Ser Ile Ala Pro Asp Ala Leu Thr Val Thr Ile Ser Glu
Ile Ala Val Lys Asp Val Thr Asp Lys Thr Gly Gly Gly Val Gln Val
Thr Asp Ser Thr Thr Gly Arg Leu Cys Met Leu Val Asp His Glu Tyr
Lys Tyr Pro Tyr Val Leu Gly Gln Gly Gln Asp Thr Leu Ala Pro Glu
Leu Pro Ile Trp Val Tyr Phe Pro Pro Gln Tyr Ala Tyr Leu Thr Val
Gly Asp Val Asn Thr Gln Gly Ile Ser Gly Asp Ser Lys Lys Leu Ala
Ser Glu Glu Ser Ala Phe Tyr Val Leu Glu His Ser Ser Phe Gln Leu
Leu Gly Thr Gly Gly Thr Ala Ser Met Ser Tyr Lys Phe Pro Pro Val

Pro Pro Glu Asn Leu Glu Gly Cys Arg Ser Thr Asp Pro Arg Glu Phe
Thr Gly Arg Arg Phe Thr Thr Ser  et/ou

Met Ser Lys Lys Ser Gly Lys Trp Trp Glu Ser Asp Asp Lys Phe Ala
Lys Ala Val Tyr Gln Gln Phe Val Glu Phe Tyr Glu Lys Val Thr Gly
Thr Asp Leu Glu Leu Ile Gln Ile Leu Lys Asp His Tyr Asn Ile Ser
Leu Asp Asn Pro Leu Glu Asn Pro Ser Ser Leu Phe Asp Leu Val Ala
Arg Ile Lys Asn Asn Leu Lys Asn Ser Pro Asp Leu Tyr Ser His His
Phe Gln Ser His Gly Gln Leu Ser Asp His Pro His Ala Leu Ser Ser
Ser Ser Ser His Ala Glu Pro Arg Gly Glu Asn Ala Val Leu Ser Ser
Glu Asp Leu His Lys Pro Gly Gln Val Ser Val Gln Leu Pro Gly Thr
Asn Tyr Val Gly Pro Gly Asn Glu Leu Gln Ala Gly Pro Pro Gln Ser
Ala Val Asp Ser Ala Ala Arg Ile His Asp Phe Arg Tyr Ser Gln Leu
Ala Lys Leu Gly Ile Asn Pro Tyr Thr His Trp Thr Val Ala Asp Glu
Glu Leu Leu Lys Asn Ile Lys Asn Glu Thr Gly Phe Gln Ala Gln Val
Val Lys Asp Tyr Phe Thr Leu Lys Gly Ala Ala Ala Pro Val Ala His
Phe Gln Gly Ser Leu Pro Glu Val Pro Ala Tyr Asn Ala Ser Glu Lys
Tyr Pro Ser  et/ou

Met Thr Ile Thr Asn Ser Asp His Met Ser Lys Lys Ser Gly Lys Trp

Trp Glu Ser Asp Asp Lys Phe Ala Lys Ala Val Tyr Gln Gln Phe Val

Glu Phe Tyr Glu Lys Val Thr Gly Thr Asp Leu Glu Leu Ile Gln Ile

Leu Lys Asp His Tyr Asn Ile Ser Leu Asp Asn Pro Leu Glu Asn Pro

Ser Ser Leu Phe Asp Leu Val Ala Arg Ile Lys Asn Asn Leu Lys Asn

Ser Pro Asp Leu Tyr Ser His His Phe Gln Ser His Gly Gln Leu Ser

Asp His Pro His Ala Leu Ser Ser Ser Ser Ser His Ala Glu Pro Arg

Gly Glu Asn Ala Val Leu Ser Ser Glu Asp Leu His Lys Pro Gly Gln

Val Ser Val Gln Leu Pro Gly Thr Asn Tyr Val Gly Pro Gly Asn Glu

Leu Gln Ala Gly Pro Pro Gln Ser Ala Val Asp Ser Ala Ala Arg Ile

His Asp Phe Arg Tyr Ser Gln Leu Ala Lys Leu Gly Ile Asn Pro Tyr

Thr His Trp Thr Val Ala Asp Glu Glu Leu Leu Lys Asn Ile Lys Asn

Glu Thr Gly Phe Gln Ala Gln Val Val Lys Asp Tyr Phe Thr Leu Lys

Gly Ala Ala Ala Pro Val Ala His Phe Gln Gly Ser Leu Pro Glu Val

Pro Ala Tyr Asn Ala Ser Glu Lys Tyr Pro Ser Met Thr Ser Val Asn

Ser Ala Glu Ala Ser Thr Gly Ala Gly Gly Gly Gly Ser Asn Ser Val

Lys Ser Met Trp Ser Glu Gly Ala Thr Phe Ser Ala Asn Ser Val Thr

Cys Thr Phe Ser Arg Gln Phe Leu Ile Pro Tyr Asp Pro Glu His His

Tyr Lys Val Phe Ser Pro Ala Ala Ser Ser Cys His Asn Ala Ser Gly

Lys Glu Ala Lys Val Cys Thr Ile Ser Pro Ile Met Gly Tyr Ser Thr

Pro Trp Arg Tyr Leu Asp Phe Asn Ala Leu Asn Leu Phe Phe Ser Pro

Leu Glu Phe Gln His Leu Ile Glu Asn Tyr Gly Ser Ile Ala Pro Asp

Ala Leu Thr Val Thr Ile Ser Glu Ile Ala Val Lys Asp Val Thr Asp

Lys Thr Gly Gly Gly Val Gln Val Thr Asp Ser Thr Thr Gly Arg Leu

Cys Met Leu Val Asp His Glu Tyr Lys Tyr Pro Tyr Val Leu Gly Gln

Gly Gln Asp Thr Leu Ala Pro Glu Leu Pro Ile Trp Val Tyr Phe Pro

Pro Gln Tyr Ala Tyr Leu Thr Val Gly Asp Val Asn Thr Gln Gly Ile

Ser Gly Asp Ser Lys Lys Leu Ala Ser Glu Glu Ser Ala Phe Tyr Val

Leu Glu His Ser Ser Phe Gln Leu Leu Gly Thr Gly Gly Thr Ala Ser

Met Ser Tyr Lys Phe Pro Pro Val Pro Pro Glu Asn Leu Glu Gly Cys

Ser Gln His Phe Tyr Glu Met Tyr Asn Pro Leu Tyr Gly Ser Ser Arg

Val Asp Leu Gln  et/ou

```
Met Thr Ile Thr Asn Ser Asp His Met Ser Lys Lys Ser Gly Lys Trp
Trp Glu Ser Asp Asp Lys Phe Ala Lys Ala Val Tyr Gln Gln Phe Val
Glu Phe Tyr Glu Lys Val Thr Gly Thr Asp Leu Glu Leu Ile Gln Ile
Leu Lys Asp His Tyr Asn Ile Ser Leu Asp Asn Pro Leu Glu Asn Pro
Ser Ser Leu Phe Asp Leu Val Ala Arg Ile Lys Asn Asn Leu Lys Asn
Ser Pro Asp Leu Tyr Ser His His Phe Gln Ser His Gly Gln Leu Ser
Asp His Pro His Ala Leu Ser Ser Ser Ser Ser His Ala Glu Pro Arg
Gly Glu Asn Ala Val Leu Ser Ser Glu Asp Leu His Lys Pro Gly Gln
Val Ser Val Gln Leu Pro Gly Thr Asn Tyr Val Gly Pro Gly Asn Glu
Leu Gln Ala Gly Pro Pro Gln Ser Ala Val Asp Ser Ala Ala Arg Ile
His Asp Phe Arg Tyr Ser Gln Leu Ala Lys Leu Gly Ile Asn Pro Tyr
Thr His Trp Thr Val Ala Asp Glu Glu Leu Leu Lys Asn Ile Lys Asn
Glu Thr Gly Phe Gln Ala Gln Val Val Lys Asp Tyr Phe Thr Leu Lys
Gly Ala Ala Ala Pro Val Ala His Phe Gln Gly Ser Leu Pro Glu Val
Pro Ala Tyr Asn Ala Ser Glu Lys Tyr Pro Ser Met Thr Ser Val Asn
Ser Ala Glu Ala Ser Thr Gly Ala Gly Gly Gly Gly Ser Asn Ser Val
Lys Ser Met Trp Ser Glu Gly Ala Thr Phe Ser Ala Asn Ser Val Thr
Cys Thr Phe Ser Arg Gln Phe Leu Ile Pro Tyr Asp Pro Glu His His
Tyr Lys Val Phe Ser Pro Ala Ala Ser Ser Cys His Asn Ala Ser Gly
Lys Glu Ala Lys Val Cys Thr Ile Ser Pro Ile Met Gly Tyr Ser Thr
Pro Trp Arg Tyr Leu Asp Phe Asn Ala Leu Asn Leu Phe Phe Ser Pro

Leu Glu Phe Gln His Leu Ile Glu Asn Tyr Gly Ser Ile Ala Pro Asp
Ala Leu Thr Val Thr Ile Ser Glu Ile Ala Val Lys Asp Val Thr Asp
Lys Thr Gly Gly Gly Val Gln Val Thr Asp Ser Thr Thr Gly Arg Leu
Cys Ser Asn
```

ou des séquences partielles de celles-ci,qui sont appropriées pour une caractérisation d'anticorps contre le parvovirus B19 dans des fluides d'analyse.

2. Peptide ou polypeptide immunologiquement actif selon la revendication 1, caractérisé en ce qu'il se présente sous forme d'une protéine de fusion, cette protéine de fusion présentant au moins une partie de la β-gnlactosidase ou de la glutathion-S-transférase.

3. Nécessaire d'essai pour la caractérisation d'anticorps contre le parvovirus B19, caractérisé en ce qu'il présente au moins un peptide ou un polypeptide immunologiquement actif selon la revendication 1, qui peut réagir avec les anticorps présents dans les fluides d'analyse, et en ce qu'il présente au moins un constituant indicateur qui permet la caractérisation de complexes de peptide immunologiquement actif et d'anticorps.

4. Nécessaire d'essai selon la revendication 3, caractérisé en ce que le constituant indicateur est un anticorps dirigé contre l'anticorps à caractériser, qui présente un marquage.

5. Nécessaire d'essai selon la revendication 4, caractérisé en ce que le marquage consiste en un isotope radioactif.

6. Nécessaire d'essai selon la revendication 4, caractérisé en ce que le marquage consiste en une enzyme, qui peut catalyser une réaction colorée.

7. Nécessaire d'essai selon la revendication 4, caractérisé en ce que le peptide ou le polypeptide immunologiquement actif est biotinylé et en ce que le constituant indicateur est de l'avidine ou de la streptavidine en liaison covalente avec une enzyme, en particulier une peroxydase.

8. Nécessaire d'essai selon l'une quelconque des revendications 4 à 7, caractérisé en ce qu'il est un nécessaire d'essai ELISA.

9. Nécessaire d'essai selon la revendication 8, caractérisé en ce qu'au moins un peptide ou polypeptide immunologiquement actif selon la revendication 1 est accouplé à des plaques de microtitrage et en ce que le constituant indicateur consiste en anticorps anti-IgG humains et/ou IgM, auxquels on accouple une enzyme catalysant une réaction colorée.

10. Nécessaire d'essai selon la revendication 8, caractérisé en ce que des anticorps monoclonaux contre des anticorps IgM humains sont accouplés à des plaques de microtitrage, et en ce que le constituant indicateur est un peptide ou un polypeptide immunologiquement actif biotinylé selon la revendication 1, qui coopère avec l'avidine ou la streptavidine par l'enzyme qui lui est reliée par liaison covalente.

11. Procédé de purification de peptides ou polypeptides immunologiquement actifs selon la revendication 1, caractérisé en ce qu'il comprend une élimination de fractions insolubles, une séparation au moyen d'une colonne Sephazell diéthylaminoéthyle, et une purification supplémentaire au moyen d'une colonne d'échangeur d'anions par HPLC dans de l'urée 8M.

12. Procédé selon la revendication 11, caractérisé en ce qu'il comprend de plus une chromatographie d'affinité.

13. Procédé selon la revendication 12, caractérisé en ce que la chromatographie d'affinité est effectuée au moyen d'une matrice de gel accouplée au glutathion.

14. Utilisation d'au moins une séquence d'ADN choisie parmi

O-1:
GTG AAT TCT GAT CAT ATG AGT AAA AAA AGT GGC AAA TGG,

O-2:
C TTC GGT CGT GAC CAC GTC CTC CCC

O-3:
G AGG AAT TCT CTG ATC ATG ACT TCA GTT AAT TCT GCA GAA GCC

O-4:
GAG GGG TGG CAC GGG AGT CGG TCC TTC GAA GAG

O-5:
G CTA CAA GCT GGG CCC CCG CAA AG

pour la caractérisation directe de l'espèce excitatrice au moyen d'une amplification d'ADN, en particulier au moyen d'une réaction de polymérase en chaîne.

15. Utilisation de peptides immunologiquement actifs selon la revendication 1 pour la préparation d'un inoculant contre des infections par le parvovirus B19.